# EUROPEAN PATENT APPLICATION

(11) **EP 1 609 872 A1**
(43) Date of publication of application: **28.12.2005**
(21) Application number: 04076880.6
(22) Date of filing: 25.06.2004
(51) Int. Cl.: C12Q 1/68

(54) **A method for selecting a gene involved in nisin resistance in bacteria**

(71) Applicant: Stichting voor de Technische Wetenschappen, 3527 JP Utrecht (NL)
(72) Inventor: Kramer, Naomi Esther, 9721 JR Groningen (NL); Kuipers, Oscar Paul, 9728 XG Groningen (NL)
(74) Representative: Winckels, Johannes Hubertus F.

(57) **Abstract**

The present invention discloses a method for selecting a gene involved with nisin-resistance in a species of bacteria comprising providing candidate genes that are differentially expressed in nisin-resistant and sensitive strains of said species and selecting from said candidate genes at least one gene that results in an altered nisin-resistance phenotype in a strain of said species, when expression of said gene is altered. The invention further provides a bacterial strain obtained by the method of the invention, and a method for fermenting dairy products with said bacterial strain. The invention also discloses cheese produced by fermentation according to the invention.

### Annex to the application documents-subsequently filed sequences listing

SEQUENCE LISTING

<110>  Stichting voor de Technische Wetenschappen

<120>  A method for selecting a gene involved in Nisin resistance in bacteria

<130>  P69230EP00

<140>  EP 04076880.6
<141>  2004-07-26

<160>  2

<170>  PatentIn version 3.1

<210>  1
<211>  28
<212>  DNA
<213>  Artificial Sequence

<220>
<223>  Primer yne forward

<400>  1
ccccggtctc ccatgattaa aatctacc                                          28


<210>  2
<211>  29
<212>  DNA
<213>  Artificial Sequence

<220>
<223>  Primer yne reverse

<400>  2
ctagtctaga ttactgacaa gaacagtcc                                         29

## Description

The invention relates to modulation of the resistance of bacteria to nisin, more in particular the invention relates to a method for selecting a gene involved with nisin-resistance in bacteria. More in particular with selecting said genes in bacteria of the genus *Lactococcus,* more in particular in *Lactococcus lactis.* The invention also relates to a method for selecting a gene involved with nisin-resistance in bacteria, more in particular in gram-positive bacteria such as for example *Bifidobacterium, Propionibacterium, Lactobacillus, Lactococcus, Listeria, Clostridium, staphylococcus, Enterococcus* and *Streptococcus* based on the expression profile of a group of selected genes. Said nisin-resistant bacterial strains can be used for the production of food products, dairy products, fermented milk products and/or fermented dairy products, like for example cheese, yoghurt or buttermilk, and also for the production of nisin in dairy products

Nisin, a lanthionine-containing peptide produced by certain strains of *L. lactis* (28), is widely used in the food industry as a safe and natural preservative (14, 27, 38) because of its antimicrobial activity against a broad range of Gram-positive bacteria, including *Listeria* species. Nisin works by disrupting the cytoplasmic membrane of a cell through pore-formation, which leads to the release of small cytoplasmic compounds, depolarization of the membrane potential and, ultimately, cell death (4, 6, 15, 17, 46, 55). Nisin uses Lipid II as a receptor for efficient pore-formation in the target membrane. The efficacy of nisin as an antimicrobial agent could seriously be compromised by the occurrence of nisin-resistance in spoilage or pathogenic bacteria. Generation of non-stable nisin-resistant (Nis^{R}) *L. lactis* under laboratory conditions is relatively easily achieved and lost by stepwise exposure to increasing concentrations of nisin (24, 39, 40). Since nisin sensitivity and the Lipid II content in the cytoplasmic membrane are not directly correlated (29), nisin-resistance is achieved through other mechanism(s).
Resistance of bacteria to nisin has been associated with an altered fatty acid composition of phospholipids (39, 40) or an altered phospholipid composition of the cytoplasmic membrane (41). Changes in the cell wall, such as over-expression of the *dlt*-operon leading to a decrease in sensitivity to mutanolysin or a thicker cell wall, have also been observed in nisin-resistant strains of *Listeria monocytogenes* and in *L. lactis* (9, 10, 29, 36). We have recently detected, that without a cell wall, a *Micrococcus flavus* strain that had acquired resistance to nisin was almost equally sensitive to nisin as its parent strain once the cell walls from both strains had been removed (29). In a spontaneous nisin-resistant strain of *L. monocytogenes* expression level of a putative penicillin-binding protein was significantly increased (22). The resistant strain was sensitive to different beta-lactam antibiotics (bind to PBPs) and showed a slight decrease in sensitivity to mersacidin, a lantibiotic, like nisin, which also binds to Lipid II (22). In *Staphylococcus aureus* was observed that, additional copies of the *dlt* operon resulted in a phenotype that was 1.6 times less sensitive to antimicrobial peptides, among which nisin, than the wild-type strain (43).
We have observed that nisin resistance can be acquired by alteration of expression of genes involved, among others, in cell membrane and/or cell wall synthesis. The present invention discloses a concise picture of the factors involved in nisin resistance. The present invention discloses nisin resistance for bacteria with as a working example, the model bacterium *L. lactis* using DNA microarrays containing amplicons of 2108 genes of *L. lactis* IL1403. Transcriptomes of *L. lactis* IL1403 and *L. lactis* IL1403 Nis^{R}, an adapted strain that is 60 times more resistant to nisin than the parent, were compared in this study. Surpisingly, we detected that although the pore formation is mediated by the binding of nisin to Lipid II, the actual resistance to nisin was mediated by a number of genes not directly related to Lipid II but instead involved in for example cell wall biosynthesis, arsenic resistance, bacitracin resistance, the Leloir pathway and genes of various putative ABC transporters.
Therefore, the present invention discloses a method for selecting a gene involved with nisin-resistance in a species of bacteria comprising providing candidate genes that are differentially expressed in nisin-resistant and sensitive strains of said species and selecting from said candidate genes at least one gene that results in an altered nisin-resistance phenotype in a strain of said species, when expression of said gene is altered.
Nisin-resistance in this specification means that a bacterium replicates and is able to withstand a MIC value that is at least 2 fold higher than a sensitive strain. A MIC value is a minimal inhibitory concentration of nisin and means that at said concentration of nisin the replication of a bacterial strain is inhibited.. Nisin resistance can also provide resistance to other lantibiotic compounds or antimicrobial peptides, like for example mersacidin or vancomycin. The method for generating nisin-resistant strains is performed as described elsewhere (29). A nisin resistance phenotype is a bacterial strain, which is resistant to nisin at a concentration, which is at least ten times higher, than the lowest concentration of nisin necessary to inhibit the growth of a sensitive strain of the same species. Sensitivity to nisin varies somewhat from strain to strain. However, a resistant strain is resistant to at least 10 x and preferably 100 x and more preferably 1000 x higher concentrations of nisin than the lowest concetration of nisin that inhibits growth of an average sensitive strain. A sensitive strain is preferably the most sensitive strain of the species. A nisin sensitive strain of *Lactococcus* is in this specification a bacterial strain that stops replicating at a low concentration of nisin, for example at a concentration of about 5 to 50 µg/ml. Alternatively, but not necessarily, nisin resistant bacteria may also show visible signs of an altered appearance, which may include a thickening of the septum of the bacteria.

In nisin resistant strains of bacteria, the expression of genes may be up or down regulated. Said up or down regulation in this specification is called differentially expression when the epression rate is 1.5 fold higher or lower than the expression rate of said gene in a wildtype species. Genes of interest, for example genes that are differentially expressed in nisin resistant strains versus wild type strains are called candidate genes in this specification.
In a preferred embodiment, the present invention also discloses a method, wherein differential expression of genes is determined by means of nucleic acid array technology. Said nucleic acid array technology is well known in the art (30, 53).
An increase in transcription was observed for genes involved in cell wall biosynthesis in the Nis^{R} strain. Excitingly, genes putatively involved in arsenic resistance, bacitracin resistance, the Leloir pathway and genes of various putative ABC transporters were also up-regulated. In a preferred embodiment, a method of the invention comprises a method wherein the involvement of a candidate gene in nisin-resistance is determined by determining that the nisin-resistance phenotype of a strain of said species changes when expression of said gene in said strain is altered.
The involvement of a selection of these genes in nisin resistance was further corroborated by analyzing knockout and over-expression mutants of these genes in *L. lactis*. A knockout mutant is a bacterial strain in which said gene is not functionally present in the genome. This can be through altering the gene or through essentially knocking out the presence of translatable RNA's in the cell. An over-expression mutant is a bacterial strain in which said gene is overexpressed. Said knockout or overexpression causes a stable resistance of the bacxterial strain. In a preferred embodiment, a method of the invention comprises a method, wherein altering expression comprises knocking out expression of said gene or over-expressing said gene in said strain.

The preferred differentially expressed genes found in microarray expression analysis of *L. lactis* IL1403 and *L. lactis* IL1403 Nis^{R}.^{*a*} are depicted in table 1 or table 2.
Statistically significantly differentially expressed (SSDE) genes found in microarray expression analysis of *L. lactis* IL1403 and *L. lactis* IL1403 Nis^{R}.^{*a*} are depicted in table 2. Statisticly insignificant does not mean that there is no biological effect of the measured difference in expression.
In a preferred embodiment, a method of the invention comprises a method wherein said candidate gene comprises a gene that is the homologue of *a Lactococcus lactis* gene depicted in table 1 or table 2. A homologue of a *Lactococcus lactis* gene is a gene having on the nucleotide level at least 85% and preferably at least 90% and more preferably at least 95% nucleotide homology. It is preferred that the homologue has a function that is at least similar if not identical to the function of the gene in the prototype formed by the gene of *Lactococcus lactis.* The homologue is preferably from a *Bifidobacterium, Propionibacterium, Lactobacillus, Lactococcus, Listeria, Clostridium, staphylococcus, Enterococcus* and *Streptococcus* preferably from Streptococcus thermophilus, or Lactobacillus delbrueckii subsp. Bulgaricus.
The homologue can be of a different genus, preferably the homologue is of a Lactococcus species. In a preferred embodiment, a method of the invention comprises said method wherein a gene involved with nisin-resistance in a species of bacteria comprises a gene that is the homologue of a *Lactococcus lactis* gene depicted in table 1 or table 2.
In an even more preferred embodiment, a method of the invention comprises a method wherein, wherein said gene is a *Lactococcus lactis* gene.
In a preferred embodiment, a method of the invention comprises a method, further comprising producing a bacterial strain wherein the expression of a gene product of a gene involved in nisin-resistance in said strain is altered, preferably through a method different from selecting in nisin containing medium, preferably the expression is altered through molecular biological means. In a more preferred embodiment, a method of the invention comprises said method, wherein said bacterial strain comprises an enhanced nisin-resistance phenotype.
The methods of the invention provide the skilled person with sufficient guidance to produce the bacterial strains. In a preferred embodiment, the invention provides a bacterial strain obtainable by a method of the invention.
In a preferred embodiment, the invention provides said bacterial strain wherein the expression of a gene product is altered of a gene that is the homologue of a *Lactococcus lactis* gene depicted in table 1 or table 2.
Presently, replication of noxious or unwanted bacteria (spoilers) in foord or dairy product production is limited by the addition of nitrate to the half-product during fermentation. Nitrate is an unwanted substance in food or dairy product; therefore, a more natural preservation is needed. Nisin is capable of reducing the growth of other bacteria in the precursor product, and is therefore a suitable and natural product in a food or dairy product such as cheese. Therefore, nisin is frequently used in the food industry, and nisin resistant bacterial strains are especially suited for use in the production of food products, dairy products, fermented milk products and/or fermented dairy products, like for example cheese, yoghurt or buttermilk. In a preferred embodiment, the invention provides a bacterial strain that is used in the production of a dairy product.
Cheese, yoghurt or buttermilk are preferred examples of dairy products, but the range of fermentated dairy products is vast. In a preferred embodiment, the dairy product is cheese.
More preferably, the invention provides a bacterial strain, wherein said strain is a *Lactococcus lactis*.
Fermentation of dairy products like for example cheese or yoghurt commences by adding a starter culture to the dairy precursor product. A starter culture in this specification means a culture comprising at least one, and preferably more bacterial strains suitable for fermenting a dairy precursor product into a dairy product like for example a cheese or a yoghurt. A precursor dairy product in this specification means a dairy product that forms the substrate for the starter culture. By fermentation of the precursor dairy product, a consumable dairy product like for example cheese or yoghurt can beformed. In a preferred embodiment, the invention provides a starter culture for fermenting a dairy precursor product comprising a bacterial strain of the invention. In a preferred embodiment, said bacterial strains are nisin-resistant bacterial strains, wherein at least one of said nisin-resistant strains is a bacterial strain of the present invention preferably obtained according to a method of the present invention.
The bacterial strains provided by the present invention and the methods as described and disclosed herein, give sufficient guidance to a skilled person to ferment a dairy precursor product. In a preferred embodiment, the invention provides a method for fermenting a dairy precursor product comprising providing said diary precusor product with a bacterial strain according the invention, or a starter culture according to the invention.
In the fermentation method as described above, additional inhibition of unwanted bacterial growth can be provided by adding nisin to the food, and preferably, dairy precursor product. In a preferred embodiment, the invention provides a method for fermenting a dairy precursor product, further comprising providing said dairy precursor product with nisin.
Preferably said nisin is produced by a bacterial strain of the invention or a bacterial strain of the starter culture. In a preferred embodiment, the invention provides a method fermenting a dairy precursor product, further comprising providing said dairy precursor product with nisin, wherein said nisin is produced by a strain according to the invention, or a starter culture according to the invention. The invention thus also provides a nisin resitant bacterial strain of the invention that is capable of producing nisin. Such a strain is able to produce more nisin because it is less inhibited by the growth inhibitory effect of nisin.

In a preferred embodiment, a method of the invention comprises a method for fermenting dairy precursor product of a food products, preferably a dairy product, more preferably a fermented milk product and/or fermented dairy products, like for example cheese, yoghurt or buttermilk.
In a preferred embodiment, the invention provides a cheese obtainable by a method of the invention as described above.
Of course, the cheese, which is produced by the methods and strains of the invention, still contains bacterial strains or parts of said bacteria (debris) as disclosed in this invention. In a preferred embodiment, the invention provides a cheese obtainable by a method of the invention, comprising a bacterial strain according to the invention, or debris thereof. Of course the type of cheese may be any type of cheese that is made by fermentation. In a more preferred embodiment, the invention provides a gouda, cheddar of emmenthaler cheese.

In another aspect the invention provides a method for selecting a nisin resistant gram positive bacterial strain comprising detecting expression of a gene that is the homologue of a *Lactococcus lactis* gene depicted in table 1 or table 2 in a gram positive bacterial strain and selecting said strain when expression of said gene is indicative for nisin resistance. Thus also natural occurring strains that are selected on the basis of expression of a gene of table 1 or table 2, to be nisin-resistant are part of the bacterial strains of the invention. Thus further provided is a bacterial strain selected for nisin-resistance on the basis of the expression of a gene that is the homologue of a *Lactococcus lactis* gene depicted in table 1 or table 2. The invention in another embodiment provides a culture comprising a nisin resistant bacterial strain of a species obtained by a method of the invention, or a bacterial strain of a species according to the invention and preferably a natural occurring strain of the invention, wherein said culture comprises nisin in a concentration that is toxic for nisin sensitive strains of said species.

**Table 1.**

| Differentially expressed genes found in microarray expression analysis of *L. lactis* IL1403 and *L. lactis* IL1403 Nis^{R}.^{*a*} | | | |
|---|---|---|---|
| *Genes* | *Average regulation (fold)* | *p-value* | *(Proposed) function* |
| *yneGH in* putative operon *yneBCDEGH* | 12.9 | 10⁻¹⁰ | *ars*CD (pFam) |
| *ysaBCD* in operon *ysaDCBA* | 10.1 | 10⁻⁸ | Bacitracin resistance prot. S. *mutans* |
| *dltC* in operon *dltABCD* | 9.4 | 10⁻⁸ | D-alanine incorporation |
| *ythAB* in operon *ythCBA* | 6.8 | 10⁻⁸ | Phage shock protein |
| *yajH* in operon *yahFGH* | 5.7 | 10⁻¹⁰ | Unknown |
| *ynhCD* after *nagA* (*ynhA*, *nagA, ynhCD)* protein | 5.5 | 10⁻⁹ | *ynhC* tellurite resistance |
| *arcAC1C2DT2* in operon *arcABC2C1TD2* | 4.0 | 10⁻⁷ | Arginine pathway |
| *yniHIJ* | 3.5 | 10⁻⁴ | Unknown |
| *glpF1* next to *pepDA* | 3.4 | 10⁻⁷ | Glycerol uptake facilitator |
| *yhcA* in operon *yhcACB* | 3.4 | 10⁻⁴ | Putative ABC transporter |
| *ybfAC* in operon *ybfADEBC* | 2.8 | 10⁻⁶ | Unknown |
| *yfhC* in operon *yfhABCGHI* | 2.6 | 10⁻⁴ | Unknown |
| *yhjA* in operon *yhcABC* | 2.3 | 10⁻⁵ | Unknown |
| *pbp2A* not in operon | 2.3 | 10⁻⁶ | Penicillin-binding protein 2 A |
| ypbG next to *ypcABCD* | 2.3 | 10⁻⁶ | Sugar kinase |
| *nagA* | 2.2 | 10⁻⁷ | *N*-acetylglucosamine-6-phosphate deacetytase |
| *yuhB* next to *pbp2A* | 2.2 | 10⁻⁴ | Putative acyltransferase |
| *yecA* in between *ptcABC* | 2.1 | 10⁻⁵ | Transcriptional regulator |
| *dnaK* | 2.1 | 10⁻⁶ | Heat shock protein |
| *butA* | 2.0 | 10⁻⁸ | Putative acetoin reductase |
| *ptcA* | 1.9 | 10⁻⁶ | Cellobiose PTS |
| *ymgGI* | 1.9 | 10⁻⁴ | Hypothetical proteins |
| *ypcAGH* (*ypcABCD-dexB-lnbA-ypcGH*) | 1.8 | 10⁻⁸ | Sugar ABC transporter |
| *grpE next to dnaK* | 1.8 | 10⁻⁶ | Heat shock protein |
| *yrjBC* in operon *yrjABCDEFGH* | 1.9 | 10⁻⁷ | Transport permease |
| pta | 1.9 | 10⁻⁷ | Phosphotransacetylase |
| kinD next to *llrD* | 1.9 | 10⁻⁷ | Histidine kinase |
| *IpiL* | 1.9 | 10⁻⁴ | Lipoate-protein ligase A |
| *rcfB* | 1.9 | 10⁻⁸ | Transcriptional regulator |
| *llrD* next to kinD regulator | 1.7 | 10⁻⁵ | DNA-binding response |
| *galKMT* in operon *galMKTE* | 1.7 | 10⁻⁴ | Galactose pathway |
| *ceo* next to *ymgF* | 1.7 | 10⁻⁴ | N₅-(1-carboxyethyl)-L-arginine metabolism, sucrose-nisin transposon ornithine synthase |
| *ispB next* to *gidB* | 1.7 | 10⁻⁵ | Heptaprenyl diphosphate synthase |
| *pydAB* Dehydrogenase | 1.7 | 10⁻⁶ | Dihydroorotate |
| *ynaCD* in operon *ynaABCDE* | 1.6 | 10⁻⁴ | ABC transporter (homology |
| to *faecalis* | | | MDR in *Enterococcus* |
| *gidBnext tp ispB* | 1.6 | 10⁻⁷ | Putative glucose-inhibited division protein |
| *sbcCD* | 1.6 | 10⁻⁵ | Exonuclease |
| *apI* | 1.6 | 10⁻⁶ | Alkaline phosphatase |
| *htrA* | 1.6 | 10⁻⁴ | Serine protease |
| *glgD* | 1.5 | 10⁻⁵ | Glycogen biosynthesis protein |
| *glpDF2* away from *glpF1* | 1.5 | 10⁻⁵ | Glycerol uptake facilitator |
| *lnbA* in between *dexB* and *ypcG* | 1.5 | 10⁻⁵ | Putative beta-*N*-Acetylglucosaminidase |

| | | | |
|---|---|---|---|
| ^{a} Values represent higher (positive) expression in the *L. lactis* IL1403 Nis^{R} strain than in the *L. lactis* IL1403 strain | | | |

**Table 2.**

| Statistically significantly differentially expressed (SSDE) genes found in microarray expression analysis of *L. lactis* IL1403 and *L. lactis* IL1403 Nis^{*R*}.^{*a*} | | | |
|---|---|---|---|
| *Genes* | *Average regulation (fold)* | *p-value* | *(Proposed) function* |
| *yeeA next to yedEF* | -4.5 | 10⁻⁹ | Maltose hydrolase |
| *pgmB* after *yeeA* | -3.4 | 10⁻⁷ | Beta-phosphoglucamutase |
| *yedEF* next to *yeeA* | -3.0 | 10⁻⁸ | β-glucoside specific PTS system |
| *ribAH* in operon *ribGBAH* | -2.7 | 10⁻⁶ | Riboflavin biosynthesis protein |
| *yvdD* in operon *yvdBCDEFG* and *pepX* | -2.3 | | Glycerol facilitator-aquaporin |
| *yriC* in operon *yriDCBA* | -2.2 | 10⁻⁵ | Xanthine/uracil permeases |
| *glnP* next to *glnQ* | -2.1 | 10⁻⁷ | Glutamine ABC transporter and substrate binding permease |
| *rpsN* alone | -2.0 | 10⁻⁶ | 30S ribosomal protein |
| *aroH* | -1.8 | 10⁻⁷ | Tyr-sensitive phospho-2- |
| dehydro- | | | deoxyheptonate aldolase |
| *hemH* | -1.7 | 10⁻⁶ | Ferrochelatase |
| *xpt* next to *pbuX* | -1.7 | 10⁻⁹ | Putative xanthine phosphoribosyltransferase |
| *phnA* in between *gapB* and *recG* | -1.6 | 10⁻⁵ | Uncharacterized Zn-ribbon-containing protein involved in phosphonate metabolism |
| *potD* (in operon, all down) | -1.6 | 10⁻⁸ | Putative ABC transporter |
| *ynbE* in between *murI* and *lysA* | -1.6 | 10⁻⁵ | Conserved hypothetical protein |
| *gidA* | -1.6 | 10⁻⁵ | Glucose inhibited division |
| protein | | | |
| *fabDG*_{*1*}*G*_{*2*}*Z*_{*1*}*Z*_{*2*} | - 1.5 | 10⁻⁵ | Fatty acid biosynthesis |
| *ybdA* | -1.5 | 10⁻⁴ | Putative transcriptional |
| regulator | | | |
| *pbuX* in between *xpt* and *ylgB* | -1.5 | 10⁻⁵ | Xanthine pennease |
| *dacA* (alone) | -1.5 | 10⁻⁶ | Extracellular protein Exp2 precursor |
| *glnR* next to *glnA* | -1.5 | 10⁻⁹ | Glutamine synthetase repressor |
| *plpABCD* | -1.5 | 10⁻⁶ | Outer membrane lipoprotein precursor |
| *rmaG* | -1.5 | 10⁻⁷ | Putative transcriptional |
| regulator | | | |

The invention is further explained in the examples, without being limited by it.

### Example 1

### Experimental procedures

*Bacterial strains, growth conditions and transformation―* The strains used in this study are presented in Table 3. All strains were grown at 30 °C in M17 broth (50) with 0.5 % glucose (GM17) with appropriate antibiotics (chloramphenicol at 5 µg/ml, and nisin at 3 mg/L when using the Nis^{R} strain). GM17 plates contained 1.5% agar. Molecular cloning techniques were performed essentially as described by Sambrook et al. (47). Restriction enzymes, deoxynucleotides and T4 ligase were obtained from Roche Diagnostics (Mannheim, Germany) and used as specified by the supplier. *L*. *lactis* NZ9000 was electroporated using a gene pulser (Biorad laboratories, Richmond, California), as described earlier (35, 57). Plasmid isolation was performed according to the method described by Birnboim and Doly (49).

*Generation of nisin-resistant variants of* L. lactis *IL1403―* A nisin stock solution was derived from nisaplin, (2.5% nisin, Aplin and Barrett, Danisco, Denmark) as described earlier (44). Nisin-resistant isogenic variants of *L. lactis* IL1403 were obtained by growing the strain in broth, while stepwise increasing the nisin concentration, as described before (29).

*Construction of the* L. lactis *IL1403 DNA microarray―* A DNA microarray of *L. lactis* IL1403 used in this study, was constructed essentially as described before (30, 53).

*RNA* isolation and *cDNA labeling―* Cells of a culture in mid-log phase (optical density at 600 nm (OD₆₀₀) of 0.7) was harvested by centrifugation at 8.000 x g for 1 minute and frozen in liquid nitrogen. Subsequently, the cDNA was obtained and labeled as described before (53). Three biological replicates in 2 dye swaps were performed under identical conditions. Hybridization was performed as described before (53) with minor changes.

*Bioinformatic Analyses―* Data were filtered to exclude artifacts, and low signal spots, using the programs Array Pro image analysis analyzer 4.5 (MediaCybernetics, Gleichen, Germany). Assuming that expression is unchanged of most of the genes, the Cy3/Cy5 ratios were normalized using a Lowess fit (56). The merged data were used for subsequent comparisons and were assessed with *MicroPreP* (54), *PreP* (11) and a t-test (25).

*Availability* ―The TIF files generated from scanning of the hybridized slides, the tables containing expression data after normalization are available at:
http://molgen.biol.rug.nl ..... The microprep software used for normalization and data-handling can be requested at:
http://molgen.biol.rug.nl/molgen/research/molgensoftware.php.

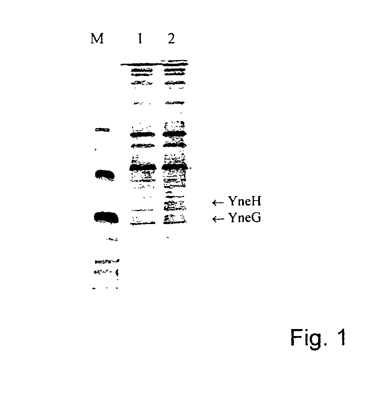

*Construction of plasmids pJK1, pFA1, pFA2 and pFA3 for expression of* yneGH *and part of the ysa-operon*―For PCR amplification of a DNA fragment carrying the *yneG* and *yneH* genes from genomic DNA of *L. lactis* IL1403, the primers *yne*-forward and *yne*-reverse, were used (Table 3). PCRs was performed with Expand polymerase (Roche Diagnostics) in accordance to the manufacturer's instructions. Amplification consisted of 1 cycle at 95 °C for 4 min, 30 cycles at 95°C for 1 min, 52 °C for 1 min and at 68 °C for 30 sec. This was followed by 1 cycle of 10 min at 68 °C. The PCR product was digested with *Bsa*I and *Xba*I (italics) and ligated into the corresponding restriction enzymes sites of pNZ8048 containing the nisA promoter, resulting in pJK1, using *L. lactis* NZ9000 as a cloning and expression host. All plasmids were introduced in *L. lactis* NZ9000 to enable nisin induction. Expression of the cloned genes, after induction with nisin for two hours, was verified using sodium dodecylsulfate polyacrylamide (17,5%) gel electrophoresis (33) (Fig. 1).

*Nisin sensitivity assays―* Sensitivity to nisin was determined essentially as described before (32) for the strains *L. lactis* NZ9000, *L. lactis* NZ9000 *ΔgalAMK, L. lactis* MG1363 and *L. lactis* MG1363 *ΔdltD* (16). For the strains of *L. lactis* NZ9000 (pNZ8048) and *L. lactis* NZ9000 (pJK1), the cells were diluted 50-fold in fresh medium. At an OD₆₀₀ of 0.2 the cells of both cultures were induced with 4 µg/L nisin for 2 h. Nisin induction of PnisA in the pNZ8048 derivatives was performed as described by de Ruyter *et al*. (12). Outgrowth in all wells was determined at the moment when wells containing cells without nisin had reached an OD₅₉₅ of 0.8, measured in an ELISA micro titer plate reader (Spectramax plus 384, Molecular Devices, Wageningen, the Netherlands). MIC values were calculated from the lowest concentration of nisin at which growth of the test strain was inhibited.

### Results

*Genome wide identification of genes involved in nisin-resistance in* L. lactis *IL1403 ―* A nisin resistant *L. lactis* IL1403 was obtained by consecutive growing in the presence of increasing concentrations of nisin in the medium. The final *L. lactis* Nis^{R} culture was colony-purified, grown in the presence of the appropriate nisin concentration and stored at -80 °C. This resulted in the isolation of an *L. lactis* strain that was 60 times more resistant to nisin than its parent (Table 4).

Global gene expression patterns *of L. lactis* IL1403 Nis^{R} and its parent were compared using the in-house developed DNA-microarrays of *L. lactis* IL1403 (53). Various genes were identified with differential expression in the Nis^{R} strain at 1.5-fold or higher(53). Of these genes, 62 were over-expressed in *L. lactis* Nis^{R} (corresponding to 2.9 % of all genes) (Table 1) and 31 were lower expressed in *L. lactis* Nis^{R} (corresponding to 1.5 % of all genes) (Table 2).

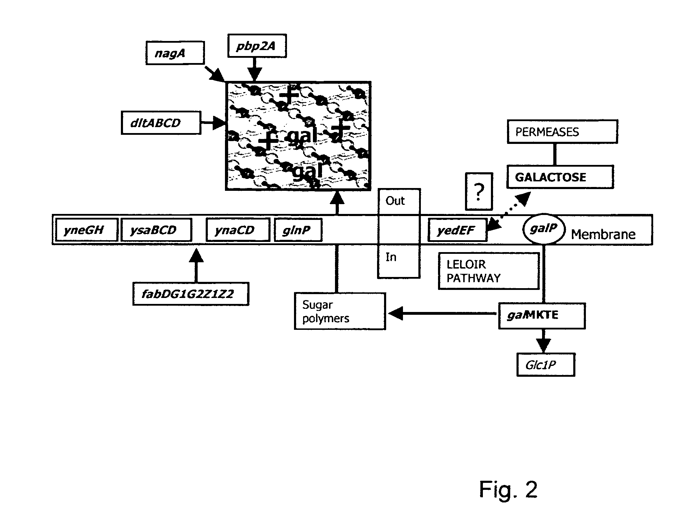

The differential expressed genes were placed in a putative nisin resistance acquiring pathway in *L. lactis* IL1403 (Fig. 2). This figure shows that the majority of the identified genes could be placed in 6 functional groups, namely (i) energy metabolism (21 genes: *galKMT, ypcAGH, ptcA, gidA, glgD, yrjBC, arcABC2T, glpD, butA, pta, ypbG, yedE* and *yeeA*), (ii) cell wall synthesis (7 genes: *dltC, dacA, galKMT, nagA* and *pbp2A),* (iii) stress response (3 genes: *htrA, dnaK, grpE*), (iv) fatty acid and phospholipid metabolism (6 genes: *fabDG1G2Z1Z2, IpiL*), (v) gene regulation (4 genes: *glnR, yecA, llrD, rmaG,),* and (vi) transport and binding proteins (17 genes: *ynaCD, ysaBC, ypcAGH, ylxcA, arcD2, glpF1F2, potD, ypcH, pbuX, yedF, ptcA* and *glnP*). One other category includes the hypothetical, unclassified or unknown proteins (36 genes: *ythAB, yajH, ysaD, yneGH, ynhCD, yniHIJ, ybfAC, yfhC, yhjA, yvhB, ybdA, ynbE, yriC, yvdD, ceo, aroH pydAB, api, sbcCD, rcfB, kinD, xpt, phnA hemH, ribAH, ispB* and *rpsN*) (not shown in figure 1). The *p*-values in a paired *t*-test, which is a t-test variant implemented in the Cyber-T software (1), ranged from 10⁻⁴ to 10⁻⁸ in the differential expressed genes. A *p*-value of 10⁻⁵ gives a chance of 4 % to be false positive(53). When genes of an entire or putative operon are found, the chance of these genes being false positive is extremely low (see figure X in the supplementary files of this article http://molgen.biol.rug.nl/publicationlnis_data/).

The identification of genes involved in cell wall synthesis, such as those in the *dlt* operon, *nagA* and *pbp2A*,supports our hypothesis (29) and existing literature that the cell wall is important in the development of nisin-resistance in bacteria. Also an increase in transcription of genes of the *gal* operon was observed. The arc operon was found 4 times over-expressed in *L. lactis* IL1403 Nis^{R}. From DNA microarray analyses (34) it has been demonstrated that these genes are under control of *ahrC*.The majority of the differential expressed genes encode hypothetical proteins. Interestingly, the amino acids sequences encoded by *yneG* and *yneH* genes share homology to those of arsenic resistance proteins in *Listeria inocua* (20), *Salmonella typhimurium* and *Escherichia coli* (48). The gene *glpF1* is 4 times over-expressed in *L. lactis* Nis^{R}, the homologue of *glpF*, which has been associated with arsenic resistance in *E. coli* (48). The products of the genes *ysaBC* have high homology to bacitracin resistance proteins in *Streptococcus mutans* (51).

*Characterizing of genes involved in cell wall biosynthesis ―* (i) *Dlt operon* ― *L. lactis* MG1363 Δ *dltD* was available in the scientific community (16). This mutants were chosen to examine whether the absence *dltD* would have an effect on nisin sensitivity. Transcription of *dltC* was 9 times higher in *L. lactis* IL1403 Nis^{R} than in *L. lactis* IL1403 after comparison on the DNA microarray and *L. lactis* MG1363 Δ *dltD* was 5-fold more sensitive to nisin than *L. lactis* MG1363 (Table 2). To examine what the impact is of mutating the genes *dltD, galAMK* and *ahrC*, we tried to make Nis^{R} derivatives of these strains by the method employed for *L. lactis* IL1403 (see Experimental Procedures section), with minor alterations. The strains were adapted to nisin through successive steps of 10, 20, 60 or 80 µg of nisin per liter. All three knock-out strains had difficulties growing in broth with a relatively low concentration (80 µg per liter) of nisin, whereas the parental strain *L. lactis* MG1363 did not. In three independent trials of obtaining a nisin resistant variant *of L. lactis* MG1363 Δ *dltD*, nisin resistance reached a plateau at 100 µg of nisin per liter, which is 12.5 times the MIC of *L. lactis* MG1363.

(ii) *pbp2A-* DNA microarray analysis showed a 2.3-fold higher expression of the gene *pbp2A* in *L. lactis* IL1403 Nis^{R} compared to its parent. No knock-out mutant was obtained, since pbp2A is an essential gene.

*Characterization of novel factors involved in the nisin resistance mechanism* ― In our own laboratory collection the strains *L. lactis* MG1363 Δ *ahrC* (34) and *L. lactis* NZ9000 (isogenic derivative of *L. lactis* MG1363 (Table 1)) Δ *galAMK* were available. These mutants were chosen to examine whether the absence of the respective genes would have an effect on nisin sensitivity of *L. lactis,* a nisin sensitivity assay was performed on each strain.

(i) *arc-* operon ― Amplicons of the *arc* genes *of L. lactis* MG1363 are also present on the *L. lactis* IL1403 DNA microarray used in this study. The arc genes *of L. lactis* IL1403 Nis^{R} hybridized to the arc genes *of L. lactis* MG1363, also showing a 4-fold over-expression. Therefore we decided to use knock-out mutants in *L. lactis* MG1363. Our DNA microarray results revealed that transcription of the *arc* operon was 4-fold increased in the nisin-resistant strain. The *ahrC* gene encodes a transcriptional regulator (repressor) of the arc operon, as was confirmed by DNA microarray analyses: deletion of *ahrC* resulted in no or only low expression of the *arc*-operon, even at a high arginine concentration in the medium (34). Deletion of *ahrC* also resulted in an increase in the expression of the arg operon of 4 to 6-fold) (34) compared to the wild-type (data not shown. *L. lactis* MG 1363 Δ *ahrC* is 5-fold more sensitive to nisin than *L. lactis* MG1363, which is consistent with our DNA microarray data (Table 2). Surprisingly, *L. lactis* MG1363 Δ *ahrC* could not grow in the presence of 100 µg of nisin per liter. *L. lactis* MG1363 Δ *ahrC* could only become approximately 10 times more resistant then its parental strain *L. lactis* MG1363, in 3 independent trials.

(ii) *gal*-operon ― Transcription of *galMKT* was 1.7 times higher in *L. lactis* IL1403 Nis^{R} than in *L. lactis* IL1403 after comparison on the DNA microarray (Table 1). *L. lactis* NZ9000 Δ *galAMK* is 2-fold more sensitive to nisin than its parent (Table 2). *L. lactis* NZ9000 Δ *galAMK* was also made resistant 150 µg of nisin per liter, which is 5.5 times the original MIC of *L. lactis* NZ9000.

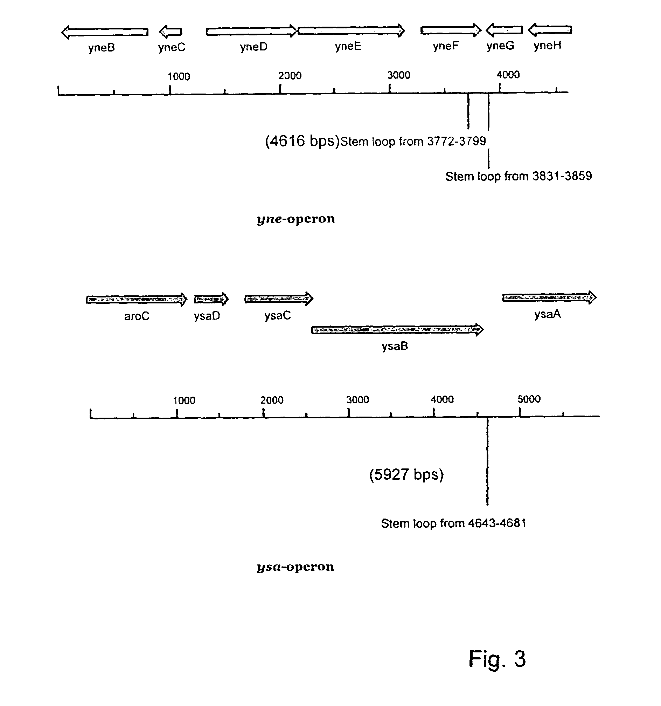

(iii) Putative arsenic resistance (*yneGH*) operon ― The genes *yneG* and *yneH* of *L. lactis* IL1403 are part of a putative operon of 7 genes (2) (Fig. 3). Both genes were 12.9-fold higher expressed in *L. lactis* IL1403 Nis^{R}. The *yneG* and *yneH* genes have no known reported function yet and as it is not known whether they are essential, an *L. lactis* strain was constructed in which both genes were over-expressed using the nisin-inducible *nisA* promoter in pJK1 (12) rather than creating a knock-out strain. Expression of *yneGH* in *L. lactis* NZ9000 pJKlwas induced by adding 10 µg nisin Z per L⁻¹. This concentration is similar to what has been described before and is about 4 times lower than the MIC *of L. lactis* NZ9000 (12). Sensitivity to nisin *of L. lactis* NZ9000 (pJK1) was compared to that of *L. lactis* NZ9000 (pNZ8048) after induction of *yneGH*. As is clear from Table 2, the over expression strain is less sensitive than its parent.

(iv) Putative bacitracin resistance (*ysaBCD*) operon ― The genes *ysaBCD of L. lactis* IL1403 are part of a putative operon of 4 genes (2) (Fig. 3). Both genes were 10.1―fold higher expressed in *L. lactis* IL1403 Nis^{R} (Table 1). Over-expression mutants were also constructed for these genes. As is shown in Table 2, the over-expression strains are less sensitive than its parent, even if only the gene *ysaD* is over-expressed.

L. lactis *IL1403 Nis*^{*R*} *is more sensitive to arsenate and bacitracin-* The resistance of *L. lactis* IL1403 Nis^{R} to mersacidin was examined as this lantibiotic also acts via specific recognition of Lipid II : it binds to the sugar-pyrophosphate moiety of Lipid II (4, 6). Mersacidin is a relatively small lantibiotic of 19 residues with no net charge (5). As no cross-resistance was observed for mersacidin, the resistance observed in *L. lactis* IL1403Nis^{R} could be specific for nisin.

The genes *ysaBCD* and *yneGH*, which are over-expressed in *L. lactis* IL1403 Nis^{R}, have homology to genes involved in bacitracin and arsenate resistance, respectively. Surprisingly, *L. lactis* IL1403 Nis^{R} is more sensitive for both bacitracin and arsenate (table 2). A Nis^{R} strain of *Streptococcus bovis* has also been shown to be more sensitive to bacitracin (37). When nisin (3000 µg/L) was added to the medium in which *L. lactis* IL1403 Nis^{R} was grown, the strain was even more sensitive for arsenate than when no nisin was present in the medium.

### Discussion

In this study we identified 93 genes belonging to different functional categories as being putatively (in-) directly involved in the acquisition of nisin-resistance in *L. lactis* IL1403 Nis^{R}. The identified genes belong to 6 functional groups, namely cell wall synthesis, energy metabolism, fatty acid and phospholipid metabolism, regulatory functions and transport and binding proteins.

Some genes involved in acquired nisin-resistance were involved in the cell wall biosynthesis, like the *dlt*-operon (9-fold more expressed in *L. lactis* Nis^{R}), which was expected since recently it was described that a *S. aureus* wild-type carrying additional copies of the *dlt* operon in *S. aureus* was less sensitive to nisin than the parent with only one copy of the *dlt*-operon on its chromosome (43). The *dlt*-operon products are involved in D-alanylation of the lipoteichoic acid (LTA) (13), by which positive charges are inserted in the LTA of the, mostly negatively charged, cell wall, respectively (43). The *dltA* and dltC genes encode the D-alanine-D-alanyl carrier protein ligase (Dcl) and the D-alanyl carrier protein (Dcp), respectively. DltB and DltD may function in transport and in the actual esterification reaction, respectively (43). The modification of LTA with D-ala introduces free amino acid groups (NH₃⁺) in the cell envelope, thus reducing the negative charge of the bacterial cell surface (42). The D-alanyl ester content in LTA is highly variable: it varies with pH, temperature and salt content in the medium. As nisin is a positively charged peptide and would be repulsed when LTA becomes more positively charged, nisin is hindered to reach the cytoplasmic membrane. This is one of the mechanisms *L. lactis* IL1403 Nis^{R} uses to acquire nisin resistance, since the *dlt* operon was 6 times more expressed compared to *L. lactis* IL1403. The direct involvement of *dlt* in the Nis^{R} phenotype was proven when the first gene of the operon was deleted: *L. lactis* MG1363 Δ *dltD* was 5 times more sensitive to nisin than *L*. *lactis* MG1363.

Our DNA microarray data revealed that transcription of *pbp2A* was 2-fold higher expressed in *L. lactis* IL1403 Nis^{R} than in *L. lactis* IL1403. In a previous study by Gravesen et al. (22) it was shown that expression of a putative penicillin-binding protein was higher in a nisin-resistant mutant of *Listeria monocytogenes.* The second stage of peptidoglycan (PG) biosynthesis, after Lipid II formation (first stage), involves strand elongation and cell wall chain cross-linking. Strand elongation is a step which is catalyzed by glycosyltransferase enzymes (GT) (52). Chain cross-linking is performed by transpeptidase enzymes (TP) by interpeptide bridge formation (19), both GT and TP are penicillin-binding proteins (PBPs). PBPs are membrane-associated molecules, present in all eubacteria and consist of two classes; the multidomain PBPs associated only with the TP activity (class B), and those bifunctional, catalyzing both GT and TP reactions (class A) (for review (21)). Class B PBPs have been found to play a unique roles in septation and regulation of cell shape, PBP2a is one of these proteins (26). These data suggest that both, the *dlt*-operon and *pbp2A*, could be part of a common nisin-resistance mechanism in bacteria. Altered expression of *pbp2A* may, additionally, affect the composition of the bacterial cell wall, thereby altering its sensitivity to cell wall-acting compounds.

Remarkably, genes involved in energy metabolism, like the *arc*-operon, which was 4-fold more expressed in *L. lactis* Nis^{R} than its parental strain. When the regulator *ahrC* is deleted, the strain becomes hardly resistant, making the *arc*-operon very important to acquire nisin resistance. Deregulation of the arginine-operon was also shown to be important in penicillin-tolerant (7).

Other genes involved in energy metabolism, such as the *gal* operon, also seem to be involved in nisin-resistance. Notably, the *gal*-operon is involved in UDP-galactose synthesis and, thus, in LTA biosynthesis (23). LTA contains a glycolipid anchor linked to the cytoplasmic membrane, a polyglycerol phosphate and various substituents (13). The substituents can vary between a hydrogen atom, D-alanyl-, α-GlcNAc- or α-Gal-. Analyzing nisin sensitivity of the *L. lactis* MG1363 Δ *galAMK* revealed that the mutant was 2 times more sensitive nisin than the wild-type strain. This result suggests that galactose is important in acquiring nisin resistance through its effect on the LTA structure. The question whether an increase/decrease in galactose in the LTA structure contributes to nisin resistance remains to be answered and whether the LTA of *L. lactis* Nis^{R} contains more galactose is currently under investigation.

Various new nisin-resistance genes were also identified, namely *yneGH* and *ysaBCD*. The gene *yneG* has homology to a trans-acting repressor *arsD* and *yneH* has homology to a arsenate reductase *arsC*. The arsenic resistance pathway of *E*. *coli* consists of *glpF* and *arsABCD*. Arsenate is taken up from the medium by the glycerol facilitator, GlpF (48). GlpF is a member of the aquaporin superfamily that transports neutral organic solutes like glycerol and urea (in *L. lactis* IL1403 there are 2 homologues: GlpF1 and GlpF2) (3). After uptake arsenate is reduced to arsenite by ArsC (16kDa). Arsenite is extruded from the cell by ArsB (As(OH)₃ →^{ArsC} As³⁺ + ATP → ^{ArsAB} H2AsO₃⁻¹ + ADP) (45). Interestingly, transcription of both *glpF1* and *glpF2* of *L. lactis* is increased. Yet, no proteins with homology to *arsA* or *arsB* were observed. Over-expressing *yneG* and *yneH* resulted in a 12.5 times more nisin resistant organism.

Homologues of S. *mutans*, namely *mbrB* (*ysaB*) and *mbrA* (*ysaC*) are involved in bacitracin resistance (51). The transporters *mbrAB* are presumed by the authors to either export a molecule that inactivates bacitracin or transports bacitracin into the cell to prevent it from binding to undecaprenol and stopping the cell wall synthesis. The genes *mbrCD* are homologous to response regulators, but their function until now in bacitracin resistance is not certain (51). The genes might be involved in transport of nisin over the membrane, to prevent it from binding to Lipid II.

A gene cluster encoding a putative beta-glucoside specific PTS system (*yedEF*) and a maltose hydrolase (*yeeA*), which were significantly reduced in the Nis^{R} strain, may additionally represent an interesting candidate for future studies. Other interesting candidates are the genes *ypcGH*, which have homology to a sugar ABC transporter. These genes are interesting to look into, since we find more genes involved in energy metabolism of *L. lactis* IL1403, like the *gal*-operon.
The genes described in this study were found in 3 independent experiments, which means they are all necessary to maintain the nisin resistant phenotype in *L. lactis* IL1403. This study is the first report that uses DNA microarray to compare global gene expression between *L.lactis* IL1403 and its nisin-resistant variant.

**Table 3:**

| Bacterial strains, plasmids and primers used in this study. | | |
|---|---|---|
| **Strain or plasmid** | **Relevant phenotype or genotype** | **Source or reference** |
| *L. lactis* IL1403 | | (8) |
| *L. lactis* IL1403 Nis^{R} | Nis^{R} | This work |
| *L. lactis* MG1363 | | (18) |
| *L. lactis* MG1363 *ΔdltD* | Derivative of *L. lactis* MG1363 carrying a deletion in *dltD* | (16) |
| L. lactis MG1363 ΔahrC | Derivative of *L. lactis* MG1363 carrying a deletion in *ahrC* | (34) |
| *L. lactis* NZ9000 | Derivative of *L. lactis* MG1363 *pepN*:: *nisRK* | (31) |
| *L. lactis* NZ9000 *ΔgalAMK* | Derivative of *L. lactis* NZ9000 carrying a deletion in *galAMK* | R. A. Neves and W. A. Pool: lab collection |
| pNZ8048 | Gen expression vector with nisin-inducible P_{*nisA*} promoter, Cm^{r} | (12) |
| *L. lactis* NZ9000 pJK1 | pNZ8048 derivative; over-expression of *yneG* and *yneH* | This work |
| *yne*-forward | 5'-CCCCGGTCTCCCATGATTAAAATCTACC -3' *Bsa*I site in underlined | This work |
| *yne*-reverse | 5'-CTAGTCTAGATTACTGACAAGAACAGTCC -3' *Xba*I site in underlined) | This work |

**Table 4:**

| MIC values for nisin of various *L. lactis* derivatives. | | |
|---|---|---|
| Strain deviation | Minimal inhibitory concentration (µg/L) | Standard |
| *L. lactis* IL1403 | 40 | |
| *L. lactis* IL1403 Nis^{R} | 3000 | |
| *L. lactis* NZ9000^{*a*} | 40 | ± 2 |
| *L. lactis* NZ9000 (pNZ8048) + induction | 40 | |
| *L. lactis* NZ9000 (pJK1) + induction | 500 | |
| *L. lactis* N29000 (pFA1) + induction | 250 | |
| *L. lactis* NZ9000 (pFA2) + induction | 350 | |
| *L*. *lactis* NZ9000 (pFA3) + induction | 280 | |
| *L. lactis* NZ9000 (Δ *galAMK*) | 27 | ± 16 |
| *L. lactis* NZ9000 (Δ *galAMK* , Nis^{R}) | 150 | |
| *L.lactisMG1363* | 40 | ± 7.5 |
| *L. lactis* MG1363 (Nis^{R}) | 3000 | |
| *L. lactis* MG1363 (Δ *dltD*) | 8 | ± 0.5 |
| *L. lactis* MG1363 (Δ *dltD*, Nis^{R}) | 100 | |
| *L. lactis* MG1363 (Δ *ahrC*) | 8 | ± 0.5 |
| *L. lactis* MG1363 ((Δ *ahrC*, Nis^{R}) | 80 | |

| | | |
|---|---|---|
| ^{*a*} Strain *L. lactis* NZ9000 (MG1363::NisRK) is an isogenic derivative of *L. lactis* MG1363. | | |

**Table 5:**

| MIC values for nisin, mersacidin, bacitracin and arsenate of *L. lactis* IL1403 and *L. lactis* IL1403 Nis^{R} | | | | |
|---|---|---|---|---|
| Strain used | Nisin (µg/L) | Mersacidin (µg/L) | Bacitracin (µg/L) | Arsenate (µg/L) |
| *L. lactis* IL1403 | 40 | 0.2 | 1.25 | 280 |
| *L. lactis* IL1403 Nis^{R} (without nisin) | 3000 | 0.2 | 0.45 | 15 |
| *L. lactis* IL1403 Nis^{R} (with nisin) | nd | nd | 0.45 | 4 |
| *L. lactis* NZ9000 (pNZ8048) | 64 | | nd | 280 |
| *L. lactis* NZ9000 (pJK1) | 800 | | nd | 1120 |

### Reference List

1. **Baldi, P. and A. D. Long.** 2001. A Bayesian framework for the analysis of microarray expression data: regularized t -test and statistical inferences of gene changes. Bioinformatics 17:509-519.
2. **Bolotin, A., P. Wincker, S. Mauger, O. Jaillon, K. Malarme, J. Wiessenbach, S. D. Ehrlich, and A. Sorokin.** 2001.The complete genome sequence of the lactic acid bacterium *Lactococcus lactis* spp. *lactis* IL1403. Genome Research 11:731-753.
3. **Borgnia, M., S. Nielsen, A. Engel, and P. Agre.** 1999. Cellular and molecular biology of the aquaporin water channels. Annual Reviews in Biochemistry 68:425-458.
4. **Breukink, E., I. Wiedemann, C. van Kraaij, O. P. Kuipers, H.-G. Sahl, and B. de Kruijff.** 1999. Use of the cell wall precursor lipid II by a pore-forming peptide antibiotic. Science 286:2361-2364.
5. **Brotz, H., G. Bierbaum, P. E. Reynolds, and H. G. Sahl.** 1997. The lantibiotic mersacidin inhibits peptidoglycan biosynthesis at the level of transglycosylation. Eur.J.Biochem. 246:193-199.
6. **Brötz, H., M. Josten, I. Wiedemann, U. Schneider, F. Götz, G. Bierbaum, and H.-G. Sahl.** 1998. Role of lipid-bound peptidoglycan precursors in the formation of pores by nisin, epidermin and other lantibiotics. Molecular Microbiology 30:317-327. Molecular Microbiology 30:317-327.
7. **Caldelari, I., B. Loeliger, H. Langen, M. P. Glauser, and P. Moreillon.** 2000. Deregulation of the arginine deiminase (*arc*) operon in penicillin-tolerant mutants of *Streptococcus gordonii.* Antimicrob.Agents Chemother. 44:2802-2810.
8. **Chopin, A., M. C. Chopin, A. Moillo-Batt, and P. Langella.** 1984. Two plasmid-determined restriction and modification systems in *Streptococcus lactis*. Plasmid 11:260-263.
9. **Crandall, A. D. and T. J. Montville.** 1998. Nisin resistance in *Listeria monocytogenes* ATCC 700302 is a complex phenotype. Applied and Environmental Microbiology 64:231-237.
10. **Davies, E. A., M. B. Falahee, and M. R. Adams.** 1996. Involvement of the cell envelope of *Listeria monocytogenes* in the acquisition of nisin resistance. Journal of Applied Microbiology **81**:139-146.
11. **de la Nava, G., S. A. F. T. van Hijum, and O. Trelles.** 2003. PreP: gene expression data preprocessing. Bioinformatics 19:2328-2329.
12. **de Ruyter, P. G. G., O. P. Kuipers, and W. M. de Vos.** 1996. Controlled gene expression systems for *Lactococcus lactis* with the food-grade inducer nisin. Applied and Environmental Microbiology 62:3662-3667.
13. **Delcour, J., T. Ferrain, M. Deghorian, E. Palumbo, and P. Hols.** 1999. The biosynthesis and functionality of the cell wall of lactic acid bacteria. Antonie van Leeuwenhoek 76:159-184.
14. **Delves-Broughton, J., P. Blackburn, R. J. Evans, and J. Hugenholtz.** 1996. Applications of the bacteriocin, nisin. Antonie van Leeuwenhoek 69:193-202.
15. **Driessen, A. J., W. van den Hooven, W. Kuiper, K. M. van de Kamp, H.-G. Sahl, R. N. Konings, and W. M. Konings.** 1995. Mechanistic studies of lantibiotic-induced permeabilization of phospholipid vesicles. Biochemistry 34:1606-1614.
16. **Duwat, P., A. Cochu, S. D. Ehrlich, and A. Gruss.** 1997. Characterization of *Lactococcus lactis* UV-sensitive mutants obtained by ISS1 transpostion. Journal of Bacteriology 179:4473-4479.
17. **Garcera, M. J., M. G. Elferink, A. J. Driessen, and W. M. Konings.** 1993. In vitro pore-forming activity of the lantibiotic nisin. Role of proton motive force and lipid composition. European Journal of Biochemistry 212:417-422. European Journal of Biochemistry 212:417-422.
18. **Gasson, M. J.** 1983. Plasmid complements of *Streptococcus lactis* NCDO 712 and other lactic streptococci after protoplast-induced curing. Journal of Bacteriology 154:1-9.
19. **Ghuysen, J. M.** 1994. Molecular structures of penicillin-binding proteins and beta-lactamases. Trends Microbiology 2:372-380.
20. **Glaser, P., L. Frangeul, C. Buchrieser, C. Rusniok, A. Amend, F. Baquero, P. Berche, H. Bloecker, P. Brandt, T. Chakraborty, A. Charbit, F. Chetouani, E. Couve, A. de Daruvar, P. Dehoux, E. Domann, G. Dominguez-Bernal, E. Duchaud, L. Durant, O. Dussurget, K. D. Entian, H. Fsihi, F. Garcia-del Portillo, P. Garrido, L. Gautier, W. Goebel, N. Gomez-Lopez, T. Hain, J. Hauf, D. Jackson, L. M. Jones, U. Kaerst, J. Kreft, M. Kuhn, F. Kunst, G. Kurapkat, E. Madueno, A. Maitournam, J. M. Vicente, E. Ng, H. Nedjari, G. Nordsiek, S. Novella, B. de Pablos, J. C. Perez-Diaz, R. Purcell, B. Remmel, M. Rose, T. Schlueter, N. Simoes, A. Tierrez, J. A. Vazquez-Boland, H. Voss, J. Wehland, and P. Cossart.** 2001. Comparative genomics of Listeria species. Science 294:849-852.
21. **Goffin, C. and J. M. Ghuysen.** 1998. Multimodular penicillin-binding proteins: an enigmatic family of orthologs and paralogs. Microbiol.Mol.Biol.Rev. 62:1079-1093.
22. **Gravesen, A., K. Sorensen, F. M. Aarestrup, and S. Knochel.** 2001. Spontaneous nisin-resistant *Listeria monocytogenes* mutants with increased expression of a putative penicillin-binding protein and their sensitivity to various antibiotics. Microbial Drug Resistance 7:127-135.
23. **Grossiord, B. P., E. J. Luesink, E. E. Vaughan, A. Arnaud, and W. M. de Vos.** 2003. Characterization, Expression and mutation of the *L lactis gal* PMKTE genes, involved in the galactose utilization via the Leloir pathway. Journal of Bacteriology 185:870-878.
24. **Harris, L., M. A. Daeschel, M. E. Stiles, and T. R. Klaenhammer.** 1989. Antimicrobial activity of lactic acid bacteria against *Listeria monocytogenes*. Journal of Food proteins 52:384-387.
25. **Hatfield, G. W., S. Hung, and P. Baldi.** 2003. Differential analysis of DNA microarray gene expression dat. Molecular Microbiology 47:871-877.
26. **Holtje, J. V.** 1998. Growth of the stress-bearing and shape-maintaining murein sacculus of Escherichia coli. Mierobiol.Mol.Biol.Rev. 62:181-203.
27. **Hurst, A.** 1981. Nisin. Advances in Applied Microbiology 23:85-123.
28. **Jung, G.** 1991. Lantibiotics: a survey, p. 1-34. *In* H.-G. Sahl and G. Jung (eds.), Lantibiotics: a survey. ESCOM Science Publishers, Amsterdam, The Netherlands.
29. **Kramer, N. E., E. Breukink, E. J. Smid, J. Kok, O. P. Kuipers, and B. de Kruijff.** 2003. Sensitivity or resistance of Gram-positive bacteria to nisin is not determined by the amount of the receptor Lipid II. to be submitted.
30. **Kuipers, O. P., A. de Jong, R. J. Baerends, S. A. F. T. van Hijum, A. L. Zomer, C. D. den Hengst, N. E. Kramer, G. Buist, and J. Kok.** 2002. Transcriptome analysis and related databases of *Lactococcus lactis*. Antonie van Leeuwenhoek 82**:**113-122.
31. **Kuipers, O. P., P. G. G. de Ruyter, M. Kleerebezem, and W. M. de Vos.** 1998. Quorum sensing-controlled gene expression in lactic acid bacteria. Journal of Biotechnology 64:15-21.
32. **Kuipers, O. P., H. S. Rollema, W. M. Yap, H. J. Boot, R. J. Siezen, and W. M. de Vos.** 1992. Engineering dehydrated amino acid residues in the antimicrobial peptide nisin. Journal of Biological Chemistry 267:24340-24346.
33. **Laemmli, U. K.** 1970. Cleavage of structural proteins during the assembly of the head of bacteriophage T4. Nature 227:680-685.
34. **Larsen, R., G. Buist, O. P. Kuipers, and J. Kok.** 2004. ArgR and AhrC are both required for regulation of arginine metabolism in *Lactococcus lactis.* Journal of Bacteriology 186:1147-1157.
35. **Leenhouts, K. and G. Venema.** 1993. Lactococcal plasmid vectors, p. 65-94. *In* K. G. Hardy (ed.), Plasmids, a practical approach. Oxford University Press, Oxford. United Kingdom.
36. **Maisnier-Patin, S. and J. Richard.** 1996. Cell wall changes in nisin-resistant variants of *Listeria innocua* grown in the presence of high nisin concentrations. FEMS Microbiology Letters **140**:29-35.
37. **Mantovani, H. C. and J. B. Russell.** 2001. Nisin resistance of *Streptococcus bovis*. Applied and Environmental Microbiology 67:808-813.
38- **Mattick, A. T. and A. Hirsch.** 1944. A powerful inhibitory substance produced by group N *streptococci*. Nature 154:551.
39. **Mazotta, A. S. and T. J. Montville.** 1997. Nisin induces changes in membrane fatty acid composition of *Listeria monocytogenes* nisin-resistant strains at 10 degrees C and 30 degrees C. Canadian Journal of Applied Microbiology 82:32-38.
40. **Ming, X. and M. A. Daeschel.** 1993. Nisin resistance of foodborne bacteria and the specific resistance responses of *Listeria monocytogenes* Scott-A. Journal of Food proteins 56:944-948.
41. **Ming, X. and M. A. Daeschel.** 1995. Correlation of cellular phospholipid content with nisin resistance *of Listeria monocytogenes* Scott A. Journal of Food Proteins 58:416-420. Journal of Food proteins 58:416-420.
42. **Perego, M., P. Glaser, A. Minutello, M. A. Strauch, K. Leopold, and W. Fischer.** 1995. Incorporation of D-alanine into lipoteichoic acid and wall teichoic acid in *Bacillus subtilis*. Identification of genes and regulation. Journal of Biological Chemistry 270:15598-15606.
43. **Peschel, A., M. Otto, R. W. Jack, H. Kalbacher, G. Jung, and F. Götz.** 1999. Inactivation of the *dlt* operon in *Staphylococcus aureus* confers sensitivity to defensins, protegrins and other antimicrobial peptides. The Journal of Biological Chemistry 274:8405-8410.
44. **Pol, I. E. and E. J. Smid.** 1999. Combined action of nisin and carvacrol on *Bacillus cereus* and *Listeria monocytogenes*. Letters in Applied Microbiology 29:166-170.
45. **Rosen, B. P.** 2002. Biochemistry of arsenic detoxification. FEBS Letters 529:86-92.
46. **Sahl, H.-G.** 1991. Pore-formation in bacterial membranes by cationic lantibiotics., p. 347-358. *In* G. Jung and H.-G. Sahl (eds.), Nisin and Novel Lantibiotics. ESCOM Science Publishers, Amsterdam, the Netherlands.
47. **Sambrook, J., E. F. Fritsch, and T. Maniatis.** 1989. Molecular Cloning: A Laboratory Manual.
   Cold Spring Harbor Laboratory, Cold Spring Harbor, New York.
48. **Sanders, O. I., C. Rensing, M. Kuroda, B. Mitra, and B. P. Rosen.** 1997. Antimenite is accumulated by the glycerol facilitator *GlpF* in *Escherichia coli*. Journal of Bacteriology 179:3365-3367.
49. **Seegers, J. F. M. L., S. Bron, C. M. Franke, G. Venema, and R. Kiewiet.** 1994.The majority of lactococcal plasmids carry a highly related replicon. Microbiology 140:1291-1300.
50. **Terzaghi, B. E. and W. E. Sandine**. 1975. Applied and Environmental Microbiology 29:807-813.
51. **Tsuda, H., Y. Yamashita, Y. Shibata, Y. Nakano, and T. Koga.** 2002. Genes involved in bacitracin resistance in Streptococcus mutans. Antimicrob.Agents Chemother. 46:3756-3764.
52. **van Heijenoort, J.** 2001. Formation of the glycan chains in the synthesis of bacterial peptidoglycan. Glycobiology 11:25R-36R.
53. **van Hijum, S. A. F. T., R. J. Baerends, H. A. Karsens, A. de Jong, N. E. Kramer, C. J. Albers, J. Kok, and O. P. Kuipers.** 2003. A generally applicable validation scheme for the assessment of factors involved in reproducibility of DNA microarray data. Nucleid Acid Research .
54. **van Hijum, S. A. F. T., J. Garcia de la Nava, O. Trelles, J. Kok, and O. P. Kuipers.** 2003. *MicroPreP*: a DNA microarray data preprocessing framework. Appl.Bioinformatics. in.
55. **van Kraaij, C., E. Breukink, M. A. Noordermeer, R. A. Demel, R. J. Siezen, O. P. Kuipers, and B. de Kruijff.** 1998. Pore formation by nisin involves translocation of its C-terminal part across the membrane. Biochemistry 37:16033-16040.
56. **Workman, C., L. J. Jensen, H. Jarmer, R. Berka, L. Gautier, H. B. Nielsen, H. H. Saxild, C. Nielsen, S. Brunak, and S. Knudsen.** 2002. A new non-linear normalization method for reducing variability in DNA microarray experiments. Genome Biology 3:research0048.1-0048.16.
57. **Zabarovsky, E. R. and G. Winberg.** 1990. High efficiency electroporation of ligated DNA into bacteria. Nucleid Acid Research 18:5912.

### Example 2

### Introduction

The development of bacterial resistance towards antibiotics is a global problem that calls for new approaches to kill microorganisms. Nisin, a positively charged lantibiotic produced by *Lactococcus lactis,* is a promising candidate because it is widely used in the food industry as a safe and natural preservative (10, 21, 27). Nisin displays a broad spectrum of activity against Gram-positive bacteria (22). Lantibiotics are peptides characterized by the presence of intra-molecular rings formed by the thioether amino acids lanthionine and 3-methyllanthionine (17, 36). Nisin kills bacteria primarily by forming pores in the cytoplasmic membrane by binding to Lipid II (2, 4), but it was recently discovered that there is no correlation between nisin sensitivity and the Lipid II content in the membrane (23). As described before, a nisin resistant *M. flavus* strain is only 1.4 times more resistant than its parental strain, without a cell wall, whereas 125 times with cell wall (23). Since the Lipid II pool is not correlated, and nisin resistant cells without cell wall have about the same sensitivity as the wild-type, the cell wall needs to be investigated.

Not only nisin, but also mersacidin and vancomycin are examples of antibiotics that inhibit the cell wall synthesis by binding to Lipid II. Mersacidin, also a member of the lantibiotics family, binds to the two-sugar-pyrophosphate moiety of Lipid II (2, 4), whereas vancomycin, a glycopeptide antibiotic, recognizes Lipid II via the L-Lys-D-Ala- D-Ala terminus of its pentapeptide side chain (19, 30). To reach the membrane and bind to Lipid II, nisin and mersacidin first have to cross the cell wall of Gram-positive bacteria, which forms a tight web around the cell (11, 13). The cell wall is made of a relatively thick multi-layered peptidoglycan sacculus, containing proteins, teichoic acids, which are wall teichoic acid (WTA) and/or lipoteichoic acid (LTA), and polysaccharides. The filtering function of the cell wall is species-dependant and is determined by the thickness and chemical structure of the wall. For example not all gram-positive bacteria have LTA and WTA, those that lack these polymers generally have similar anionic ones (34, 39), like in *Micrococcus luteus,* lipomannan is found as an LTA substitute (33). To date no reports have been made about *L. lactis* WTA, probably no WTA is present in this strain. LTA contains a glycolipid anchor linked to the cytoplasmic membrane, a polyglycerol phosphate and various glycosyl substituents (9). The substituents can vary between are H-, D-alanyl-, α-GlcNAc- or α-Gal-. The synthesis of D-alanyl-LTA requires 4 proteins that are encoded by the *dlt* operon (9). This operon incorporates positive charges in the LTA of a mostly negative charged cell wall (31). The synthesis of α-Gal-LTA requires proteins encoded by the *gal* operon. The gene *galE* is involved in UDP-galactose synthesis and, thus, in LTA biosynthesis (18)

Insights into the way bacteria develop nisin-resistance and the basis of the differences in nisin-sensitivity are of major importance for future applications of nisin or nisin variants. The new insights that the cell wall, and not Lipid II, is a major determinant in nisin sensitivity prompted us investigate the origin of resistance of bacteria for nisin, and the possible role of the cell wall. For this purpose, two different species of Gram-positive bacteria, namely *M. flavus* and *L. lactis,* and their isogenic nisin resistant variants were selected. Both stains are very sensitive to nisin (MIC = 20 µg/L for *M. flavus* and 50 µg/L for *L. lactis),* but can become very resistant, 125 times and 60 times more resistant, respectively. This study shows that nisin resistant variants become less sensitive to cell wall hydrolase mutanolysin. The article also reveals that cell walls bind nisin and cell walls of the nisin resistant variants bind even more nisin than its parental strains. The LTA structure *of L. lactis* IL1403 was compared to *L. lactis* IL1403 Nis^{R} and *L. lactis ΔdltD,* revealing more D-alanyl ester and galactose substitution in in the LTA structure of the nisin-resistant variant and a decrease of D-alanyl ester in the LTA structure *of L. lactis ΔdltD*.

### Materials and Methods

*Bacterial strains, growth conditions* ― The strains used in this study are presented in table 6 All strains were grown at 30 °C in M17 broth with 0.5 % glucose (GM17) with appropriate antibiotics (chloramphenicol at 5 µg/ml and nisin at 3 mg/L when using the Nis^{R} strain). GM17 plates contained 1.5% agar.

*Generation of nisin-resistant variant of L.* lactis *IL1403―* A nisin stock solution was derived from nisaplin, (2.5% nisin, Aplin and Barrett, Danisco, Denmark) as described earlier (32). Nisin-resistant isogenic variants of *L. lactis* IL1403 were obtained by growing the strains in broth, while stepwise increasing the nisin concentration, as described before (23).

### Determination of the Minimal Inhibitory Concentration

*M. flavus* and *L. lactis* were grown overnight at their optimum growth conditions. The Nis^{R} variants were grown in the presence of nisin (3 mg/L). The MIC values were estimated essentially as described before (23).

*Cell wall isolation and enzymatic digestion of cell walls ―* Cell wall fractions were prepared from overnight cultures of *M. flavus, M. flavus* Nis^{R}, *L. lactis* and *L. lactis* Nis^{R} as described before, with a few alterations (38). Cells were harvested by centrifugation at 4600 x g for 15 min and resuspended in 1 ml of distilled water. To every ml of cells, 500 mg of glass beads (50-105 µm) was added. Cells were broken using a Fast Prep (BIO101, Carlsbad, CA) in 3 cycles at speed setting 6.5 for 45 sec. Cellular debris was spun down at 3000 x *g* for 30 min. Cell wall fragments present in the supernatant were harvested by centrifugation at room temperature for 15 min at 20,800 x g, dried and resuspended in distilled water to a concentration of 10 µg/µl, and stored at -20 °C until use.

The sensitivity of bacterial strains to cell wall hydrolytic enzymes was determined as described earlier (28). Strains were harvested in the mid-exponential growth phase (at an OD₆₀₀ of 0.5-0.8) and boiled for 15 min to inactivate autolysins. Samples (1 ml) were centrifuged at 18,000 x g for 20 min after which the pellets were resuspended in 50 mM NaH₂PO₄ (pH 6.5) to an OD₆₀₀ of 0.4. Mutanolysin (Sigma) was added to a final concentration of 20, 50, and 100 U/ml, respectively. The decrease in optical density at room temperature was measured during 90 min (41).

*Carboxyfluorescein leakage assay* ― Large uni-lamellar lipid vesicles consisting of 1,2-dioleoyl-*sn*-glycero-3-phosphocholine (DOPC) and 0.1 mol % Lipid II were made as described by Breukink et al. (1)following the protocol by Sims et al. (37) with an internal buffer composed of 50 mM carboxyfluorescein (CF; Molecular Probes) in 25 mM MES-KOH (pH 6.0), 100 mM K₂SO₄. Unincorporated CF was removed using a Sephadex G-50 spin column equilibrated in 50 mM MES-KOH (pH 6.0) and 100 mM K₂SO₄. Cell wall fragments were incubated with nisin at room temperature for 10 min prior to mixing with CF filled vesicles and the increase in the fluorescence intensity at 515 nm (excitation at 492 nm) due to nisin induced leakage of CF was recorded in time. The nisin-induced CF leakage was expressed relative to the total amount of CF released after lysis of the vesicles by addition of 10 µl of a 20% Triton X-100 solution.

*LTA purification procedure and structure analysis ― L. lactis* IL1403, *L. Lactis* IL1403 Nis^{R} and *L. lactis* MG1363 *ΔdltD* were cultured overnight at 30 °C in 1 liter flasks, glucose was added to a final concentration of 0.5 %. Twenty grams cells of each strain were harvested and resuspended in 0.1 M citrate buffer pH 4.75. Hereafter the cells were disrupted with a sonifier, 3 times 5 minutes at speed 6. The LTA purification is done essentially as described before (29), with minor modifications. The disrupted cells were mixed with an equal volume of *n*-butanol (Merck, Darmstadt, Germany) and centrifuged at 13,000 g for 30 minutes. The aquatic phase was lyophilized, filtered using a 0.20 µm membrane filter NC 20/20 (Schleicher and Schuell, 's Hertogenbosch, the Netherlands) and resuspended in chromatography start buffer (15% n-propanol in 0.1 M ammonium acetate pH. 4.7) and centrifuged at 45,000 g for 1 hour. The supernatant was subjected to fast performance liquid chromatography (FPLC) on octyl-sepharose, with a 15 to 60 % propanol gradient (Amersham Pharmacia Biotech). The phosphor content of the LTA was estimated according to the protocol of Rouser (35). Nuclear magnetic resonance (NMR) experiments were performed at 640.13 MHz (¹H) and 300 K, and were essentially done as described before (29).

*Electron Microscopy pictures ―* Cells of *L. lactis* IL1403 or *L. lactis* IL1403 Nis^{R} were harvested by centrifugation for 1 minute at 10000 x g. Cells were fixated in 3% Gluraaraldehyde 0.1M Sodium-cacodylate buffer pH. 7.2 (CAC) and kept on ice for 2 hours. Subsequently, the cells were washed three times in buffer CAC. Hereafter fixated in Osmium tetroxide in CAC at RT and washed three times with mQ. Contrast coloration was performed with 0.4% Uranylacetate and re-hydrated with an ethanol series. Than the cells were embedded in Epon synthetic resin and sliced on a LKM ultra-microtome The sliced cells were viewed in a Philips CM10 electron microscope (FEI Company, Eindhoven, The Netherlands).

### Results

*Isolation of Nis*^{*R*} *variants of* M. flavus *and* L. lactis ― By adding nisin to the medium, as described in the experimental section, a nisin resistant variant of *L. lactis* (Nis^{R}) was obtained that could grow in the presence of 3 mg of nisin per liter, which is 60 times the MIC value for the *L. lactis* parental strain. A nisin resistant *M. flavus strain* (Nis^{R}) could grow in the presence of 2.5 mg nisin/L which is 125 times the MIC value of the wild-type strain (Table 7). Nisin was always added to the growth media to maintain resistance in all experiments.

*Nisin-specific resistance* ― To get information on the specificity of the resistance, cross-resistance of the Nis^{R} strains to mersacidin and vancomycin, antibiotics, that also act via specific recognition of Lipid II, was examined. No change in MIC was observed for vancomycin and mersacidin in the nisin resistant strain (9).

L. lactis *Nis*^{*R*} *and* M. flavus *Nis*^{*R*} *strains are less sensitive to the cell wall hydrolase mutanolysin ―* The most obvious candidate for shielding Lipid II in the membrane from nisin is the bacterial cell wall. Previous studies have shown that alterations occur in the cell wall of nisin resistant strains of various bacteria (7, 8, 26, 32, 41). To examine whether this is also the case for the Nis^{R} strains isolated in this study, we compared the sensitivity of cells of *M. flavus* and *L. lactis* and their isogenic nisin resistant variants for the cell wall hydrolase mutanolysin. Mutanolysin an *N*-Acetyl muramidase (5) is a muralytic enzyme that cleaves the *N*-acetylmuramyl-β(1-4) N-acetylglucosamine linkage of the bacterial cell wall polymer (25).

Upon incubation with 100 U/ml of mutanolysin a slower decrease of the OD₆₀₀ was observed in *M. flavus* Nis^{R} and *L. lactis* Nis^{R} compared to the respective parent strains (Table 8). This corresponds to less efficient degradation of the cell wall of both resistant strains compared to their respective parents. This result showss that the acquisition of resistance to nisin in the *L. lactis* and *M. flavus* Nis^{R} strains is associated with changes in the cell wall structures.

*Isolated cell walls capture nisin* ― The possibility that nisin binds to the cell wall was further investigated by incubating nisin with isolated cell walls. The remaining nisin activity towards Lipid II-containing vesicles was tested using a CF leakage method (2, 42). CF is quenched inside the membrane vesicles, but fluoresces upon release after pore formation by nisin. The influence of 10 nM of nisin, pre-incubated with cell walls of *M. flavus, M. flavus* Nis^{R}, L. *lactis* or *L. lactis* Nis^{R}, on vesicle stability was examined (Table 9). Addition of 10 nM nisin, pre-incubated in the absence of cell walls, to the membrane vesicles resulted in a great amount of CF leakage. After preincubation with cell walls, the leakage drops considerably. The CF leakage drops even more in the Nis^{R} variants.

This shows that nisin, in fact binds already considerable to the cell wall of the wild type strain. However, for the Nis^{R} variants, significantly more nisin is bound to its isolated cell walls, than its parental strains. These data show that the shielding effect of the cell wall for the Nis^{R} strains is in part caused by direct sequestering of nisin to prevent it from reaching the membrane.

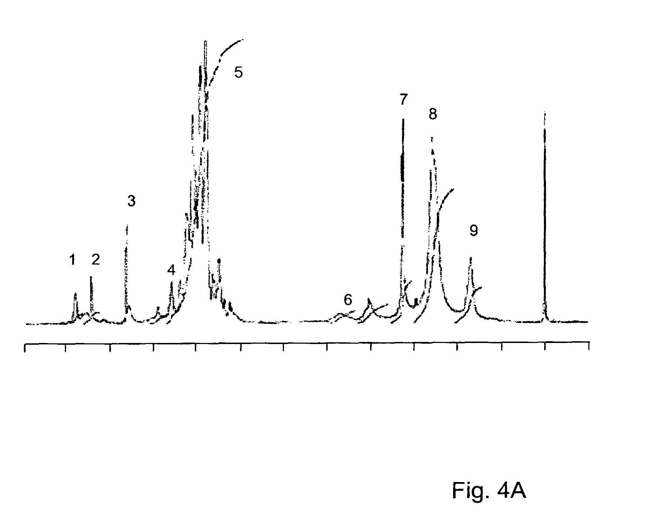

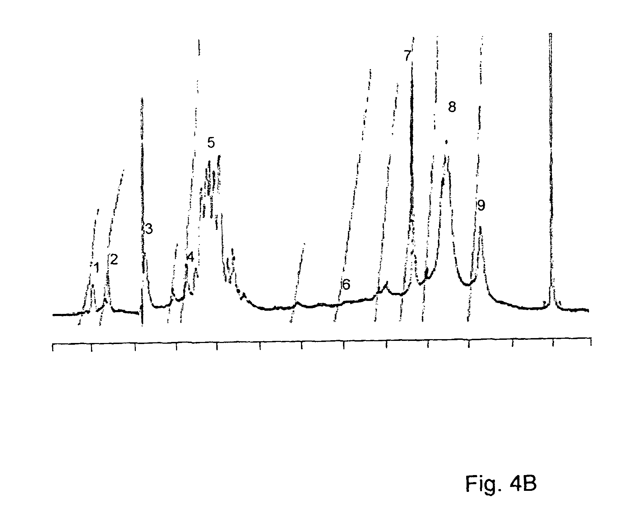

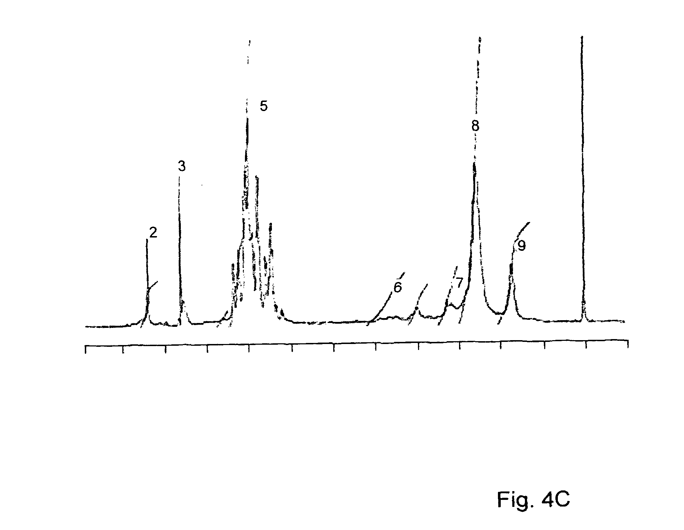

*LTA of* L. lactis *Nis*^{*R*} *is severely changed* ― To test the influence of the LTA on nisin resistance, LTA was purified from *L. lactis* IL1403 grown on glucose and galactose, *L. lactis* IL1403 Nis^{R} and *L. lactis* MG1363 *ΔdltD. L. lactis* MG1363 *ΔdltD* is a deletion derivative available in the scientific community, which is 5 times more sensitive for nisin than its parental strain. Figure 4 presents the resonance signal intensities of the D-alanine, galactose, glycerol and fatty acid fractions, of the LTA obtained with NMR. The resonance signal intensities allow for quantification of the substituents and the average of the glycerophosphate chain length as described before (29). The integral ratio of glycerol and the α-methylene group of the membrane anchor identified an average backbone length of 15.5 for *L. lactis* IL1403 on glucose (C₁₅-C₁₆), whereas the backbone length was only 7 for *L. lactis* IL1403 Nis^{R} (C₁₁- C₁₂). When *L. lactis* IL1403 grew on galactose the backbone length shortened to about 8 (C₁₁- C₁₂). In *L. lactis* MG1363 *ΔdltD* (C₁₈- C₁₉) the average backbone length was about 12. The percentage of glycerophosphate units that were esterified with D-alanine was about 24 %, 13 % with galactose and the 63% had no substituent in *L. lactis* IL1403 on glucose. Whereas when grown on galactose the percentage of glycerophosphate units that were esterified with D-alanine was about 8 %, 50 % with galactose and the 42% had no substituent. In *L. lactis* IL1403 Nis^{R} 41 % D-alanine, 28 % galactose was incorporated in the glycerophosphate units, leaving 31 % units without substituent. For *L*. *lactis* MG1363 *ΔdltD* only 4 % glycerophosphate units were esterified with D-alanine, 46 % with galactose and 50 % was un-substituted.

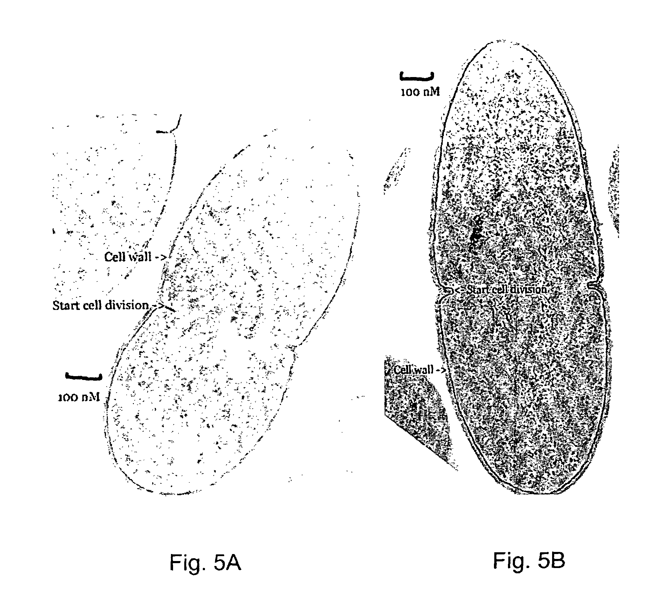

*The septum of* L. lactis *Nis*^{*R*} *is thicker than the septum of* L. lactis― The cells of *L. lactis* Nis^{R} are less sensitive to the cell wall hydrolase mutanolysin, indicating a cell wall change in this strain. Therefore electron microscope (EM) pictures were taken to see whether these changes could be visualized (Fig. 5 A and B). No difference in cell wall thickness was observed between the nisin resistant and the parental strain, as shown in table 10, yet the cell wall appears to be more roughly shaped in *L. lactis* Nis^{R} compared to the wild-type. Surprisingly, a difference in thickness of the septum was observed in the *L*. *lactis* strains, the septum *of L. lactis* Nis^{R} was thicker than its parental strain (table 10).

### Discussion

The MIC-values of the wild-type strains of *M. flavus* and *L. lactis* are in the concentration range at which nisin is highly dependant on the presence of Lipid II for its activity (2, 42). Therefore, the resistance to nisin in the Nis^{R} strains most likely still originates from a Lipid II-related mechanism. Since the Lipid II content is not correlated to nisin sensitivity (23), at least three possibilities for the nisin resistance mechanism can be envisaged. First, an increased turnover of the sugar moieties to form peptidoglycan may take place, which would decrease the possibility of nisin to bind to Lipid II. Indeed, a putative gene encoding a penicillin binding protein has been found to be more expressed in a spontaneous nisin resistant strain *of L. monocytogenes* (16) and in *L. lactis* IL1403 (24). Second, it is possible that another molecule is exported out of the cell, preventing nisin from binding to the Lipid II molecule (direct shielding). Third, nisin could be prevented from reaching the cytoplasmic membrane and Lipid II, due to changes in the cell wall, like a more positive charge (*dlt* operon) or a thicker cell wall (indirect shielding).

To get an indication about the latter possibility in nisin resistant *M. flavus* and *L. lactis* strains, we examined cross-resistance to other peptides, which also bind to Lipid II, namely mersacidin and vancomycin. Mersacidin binds to two sugar-pyrophosphate moieties of Lipid II (2, 4) and is a relatively small lantibiotic of 19 residues with no net charge (3). Vancomycin is about the same size as mersacidin and carries one positive charge. It binds to the pentapeptide side chain L-Lys-D-Ala-D-Ala moiety of the Lipid II molecule. Since they are both smaller and less charged than nisin, they are expected to cross the cell wall more easily, with reduced interference by electrostatic interactions. Because both peptides are less charged than nisin, the cell wall composition/charge could play a role in nisin resistance.
The results with the cell wall hydrolyzing enzyme mutanolysin indeed point to changes in the cell wall composition. Mutanolysin catalyzes the hydrolysis of the β-1,4 glycosidic bond between *N*-acetylmuramic acid and *N* acetylglucosamine in peptidoglycan. Both nisin resistant strains are less sensitive to the action of mutanolysin than their respective parental strains. Previously, nisin resistance in *L. monocytogenes, S. thermophilus* and *L. lactis,* has also been reported to be accompanied by altered sensitivity to cell wall hydrolyzing enzymes, such as mutanolysin (7, 8, 26, 41). This could either be caused by an increase in the degree of cross-linkage in the cell wall leading to a decreased mutanolysin activity. Or by the formation of a thickened cell wall, as has been observed by electron microscopy studies of a Nis^{R} strain of *Listeria innocua* (26). The similar outcome of all these studies, including ours, indicates that cell wall changes are generally associated with the acquisition of a nisin resistant phenotype.

Assuming that the observed changes in the cell wall are indeed responsible for the observed nisin resistance, removal of the cell wall should render the bacteria again sensitive to nisin. This is the case as has been described before, protoplasts of *L. lactis* Nis^{R} and *M. flavus* Nis^{R} were only slightly less sensitive to nisin than its parental strains (23). These experiments directly demonstrate that changes in the cell wall are the main cause of the nisin-resistant phenotype, suggesting that the cell wall is a major determining factor in nisin resistance. The CF leakage assay results showed that nisin binds more effectively to the cell wall of *M. flavus* Nis^{R} and *L. lactis* Nis^{R} than the cell walls of their parental strains. This indicates that at least part of Lipid II shielding effect is caused by direct binding of nisin to the cell wall, most likely via electrostatic interactions of the relatively high positively charged nisin with the negatively charged cell wall.

While electrostatic interactions probably cause nisin to bind to the cell wall, the LTA needs to be investigated while LTA is responsible for the variation of the cell wall charge from negative to less negative by incorporating D-alanyl esters. LTA consists of a glycolipid anchor linked to the cytoplasmic membrane, a polyglycerol phosphate and various glycosyl substituents, which are H-, D-alanyl-, α-GlcNAc- or α-Gal-. (9). Synthesis of D-alanyl moiety in the LTA is performed by the *dlt* operon, by which positive charges are inserted in the LTA of the mostly negative charged cell wall (31). The LTA analysis by NMR revealed that a 60 times more resistant *L. lactis* IL1403 strain contained more D-alanyl ester and more galactose substituents in the LTA than its parental strain. It also revealed *L. lactis* MG1363 *ΔdltD,* which is 5 times more sensitive than *L. lactis* MG1363, only has 4 percent D-alanyl ester. These results clearly indicate that the charge of the LTA is of major importance to nisin resistance. More positively charged cell walls will repulse the positively charged nisin and prevent it from reaching Lipid II in the membrane.

The results it also revealed galactose to be an important substituent in LTA for nisin resistance. Galactose might be responsible for tightening the cell wall structure. Another interesting observation from the NMR data is that the backbone length and the amount of C atoms is severely reduced in *L. lactis* Nis^{R} compared to its parental strain. This phenomenon could explain why the entire cell wall of *L. lactis* Nis^{R} still binds more nisin, since the cell wall thickness has not changed. Since LTA is attached to the cell wall and is shorter compared to the parental strain, a gradient in charge might appear in the cell wall, making the outside more negatively charged than the inside of the cell wall. This way the outside of the cell wall binds nisin while the inside prevents nisin from binding to Lipid II in the membrane. The reduction in length of the LTA might also explain the rougher appearance of the cell wall surface. The cell wall surface might be less solid in *L. lactis* Nis^{R} than the cell wall surface in the parent.

Septum thickness, however, doubled in size in *L. lactis* Nis^{R}. An explanation for this phenomenon could be penicillin-binding proteins (PBPs). PBPs catalyze peptidoglycan (PG) (40) strand elongation (transglycosylase activity) and regulate PG side chain cross-linking (14). PBPs are membrane-associated molecules, present in all eubacteria and consist of two classes; the multidomain PBPs associated only with the chain cross-linking activity (class B), and those bifunctional, catalyzing both transglycosylase activity and chain cross-linking reactions (class A) (15). Class B PBPs have been found to play a unique roles in septation and regulation of cell shape (20) and PBP2A, part of the class B PBPs was shown to be higher expressed in *L. lactis* Nis^{R} (24). This could explain the difference in septum thickness in *L. lactis* Nis^{R} compared to its parent.
The acquired Nis^{R} phenotype that we observed in *M. flavus* and *L. lactis* is most likely a combination of different effects that add up to a final high nisin resistance level, in which changes in the cell wall play the most prominent role. Previous studies have reported changes in the phospholipid composition in nisin resistant strains (7, 28). The overall content of negatively charged lipids decreased to some extent. This was also the case in our strains (data not shown). In conclusion, shielding of the membrane is a major general defense mechanism of Gram-positive bacteria against nisin.

**Table 6:**

| Bacterial strains used in this study | |
|---|---|
| **Strains** *Micrococcus flavus* NIZO B423 | **Source or reference** NIZO Food Research |
| *Micrococcus flavus* NIZO B423 Nis^{R} | This work |
| *L. lactis IL1403* | (6) |
| *L. lactis IL1403* Nis^{R} | This work |
| *L. lactis* MG1363 Δ*dltD* | (12) |
| *L. lactis* MG1363 *ΔgalAMK* | Neves, A.R., Pool, W. A.: lab collection |

**Table 7:**

| MIC values for nisin, vancomycin and mersacidin of nisin-sensitive and resistant strains of *L*. *monocytogenes* and *M. flavus*. | | | |
|---|---|---|---|
| Minimal inhibitory concentration (µg/L)^{a} | | | |
| Strain | Nisin | Vancomycin | Mersacidin |
| *M. flavus* | 20 | 450 | 770 |
| *M. flavus* Nis^{R} | 2,500 | 450 | 2,250 |
| *L. lactis* | 40 | | |
| *L. lactis* Nis^{R} | 3,000 | | |
| *L. lactis* MG1363 | 40 | nd | nd |
| *L. lactis* MG1363 Δ*dltD* | 8 | nd | nd |
| *L. lactis* MG1363Δ*galAMK* | 27 | nd | nd |

| | | | |
|---|---|---|---|
| ^{a}Standard errors were less than 15% of the given values. nd = not done | | | |

**Table 8:**

| Degradation of cell walls from nisin sensitive and resistant *L lactis* and *M. flavus* | |
|---|---|
| **Strain** | **A OD**_{**600**}**/min** |
| *M. flavus* | 0.1 |
| *M. flavus* Nis^{R} | 0.04 |
| *L. lactis* IL1403 | 0.012 |
| *L. lactis* IL1403 Nis^{R} | 0.009 |
| * 1 ml of cells from a culture at OD₆₀₀ of 0.4, was incubated with 100 units mutanolysin for i minute. Degradation of the cell wall was monitored as a drop in OD₆₀₀. | |

**Table 9:**

| Nisin-induced carboxyfluorescein leakage from DOPC-0.1% Lipid II vesicles. | | | | |
|---|---|---|---|---|
| Cell walls µg ** | *M. flavus *** | *M. flavus* Nis^{R}** | *L. lactis* IL1403** | *L. lactis* IL1403 Nis^{R} |
| 0 | 39.5 | 39.5 | 53 | 53 |
| 2 | 14.5 | 7.5 | 43 | 32 |
| 5 | 10 | 1.5 | 30 | 22 |
| 10 | 9 | 0.1 | 21 | 15 |

| | | | | |
|---|---|---|---|---|
| * based on 2 independent measurements. | | | | |
| * % release | | | | |

**Table 10:**

| Thickness of the cell wall and the septum in *L. lactis* and *L. lactis* Nis^{R}. | | |
|---|---|---|
| Strain | Cell wall thickness* | Septum thickness* |
| *L. lactis* | 17 nm ± 1 | 5 nm ± 2 |
| *L. lactis* Nis^{R} | 17 nm ± 1 | 11 nm ± 2 |

| | | |
|---|---|---|
| * Based on 10 measurements | | |

**Figure 1: SDS-PAGE (17.5%) of** ***L. lactis*** **NZ900 (pNZ8048) with** ***L. lactis*** **NZ9000 (pJK1).** Lane 1 represents *L. lactis* NZ9000 (pNZ8048) and lane 2 *L. lactis* NZ9000 (pJK1). The arrows point out the proteins YneH and YneG, respectively.

**Figure 2: Putative nisin resistance pathway.** Genes higher expressed in *L. lactis* Nis^{R} are red and lower expressed are green. The putative nisin resistance pathway can be divided into 6 major functional groups. First the ABC transporters a total of 7 genes, namely *ysaBC, ypcGH, ynaCD* and *glnP,* they might be involved in pumping nisin into the cell to prevent it from binding to Lipid II, which is present in the membrane. Energy metabolism genes; like *galMKTE* and *ypcGH* (also an ABC transporter) are higher expressed in the nisin resistant strain and other genes involved in energy metabolism (*yedEF* and *yeeA*) are lower expressed in the nisin resistant strain. It as a possibility that if one of the energy metabolism genes is higher expressed, resulting in others genes which are lower expressed. As a last group, genes involved in the cell wall synthesis are higher expressed in *L. lactis* Nis^{R} (*nagA, pbp2A* and the *dlt* operon). A highly expressed *dlt* operon accounts for a more positive charge in the lipoteichoic acid, making the cell wall also more positive. Nisin is also positively charged and this way repulsed by the cell wall. Than only one is involved in the cell membrane synthesis, namely *fabZ2*, which is lower expressed in *L. lactis* Nis^{R}, it is involved in de-saturation of the phospholipids in the membrane. When the phospholipids are more saturated, nisin can not insert into the membrane. Other categories are a total of 7 which contain *glpF1, glpF2, arcABC2D2* and *rpsN*. And the last group contained the hypothetical, unclassified or unknown proteins (9 genes: *ythAB, yajH, ysaD, yneGH, yrjCDE)*. In what way the last two groups are involved in the acquisition if nisin resistance, has to be investigated.

**Figure 3: The** ***yne-*** **and** ***ysa*****-operon.** The *yne* operon in *L. lactis* IL1403 consists of 7 (putative) genes. The gene *yneB* has homology to an esterase/lipase. The gene *yneC* is a hypothetical protein. *yneD* has homology a short-chain dehydrogenase/oxidoreductase. *yneE* is a putative transcriptional regulator. *yneF* is a hypothetical cytosolic protein and *yneG* and *yneH* have homology to the arsenic resistance genes *arsC* and *arsD*. There are 2 stem loops present in between the genes *yneF* and *yneG*, pointing to different operons. The *ysa* operon in *L. lactis* IL1403 consists of 4 (putative) genes. The gene *ysaA* has homology to membrane proteins. The gene *ysaB* has homology to ABC transporter, permease protein. The gene *ysaC* has homology to an ABC transporter, ATP-binding protein and *ysaD* is a hypothetical protein.

**Figure 4: NMR of** ***L. lactis*** **IL1403,** ***L. lactis*** **IL1403 Nis**^{**R**} **and** ***L. lactis*** **MG1363** ***ΔdltD*****.** ¹H NMR spectrum (600 MHz, 300 K) of LTA obtained after butanol extraction. A represents the NMR *of L. lactis* IL1403, B represents *L. lactis* IL1403 Nis^{R} and C represents *L. lactis* MG1363 *ΔdltD*. Number 1 represents Gro-2-CH-(D-ala), number 2 represents galactose 1-H, number 3 represents deuterium-oxide, number 4 represents D-ala-α-H, number 5 represents the glycerol peaks, number 6 represents the CH₂-α fatty acids, number 7 represents D-ala-β-H, number 8 represents CH₂-ω fatty acids and number 10 represents CH₃ fatty acids.

**Figure 5: Electron microscopy pictures of** ***L. lactis*** **and** ***L. lactis*** **Nis**^{**R**}**.** Figure A represents a bacterial cell of *L. lactis* and figure B a bacterial cell of *L. lactis* Nis^{R}. The arrows indicate the cell wall and the cell division sites in both strains. Cell wall thickness is 17 nm in both strains. Septum thickness is 11 nm in *L. lactis* Nis^{R} and 5 nm in *L. lactis.*

### Reference List

**1. Breukink, E., H. E. van Heusden, P. J. Vollmerhaus, E. Swiezewska, L. Brunner, S. Walker, A. J. Heck, and B. de Kruijff.** 2003. Lipid II is an intrinsic component of the pore induced by nisin in bacterial membranes. Journal of Biological Chemistry 278:19898-19903.
2. **Breukink, E., I. Wiedemann, C. van Kraaij, O. P. Kuipers, H.-G. Sahl, and B. de Kruijff.** 1999. Use of the cell wall precursor lipid II by a pore-forming peptide antibiotic. Science 286:2361-2364.
3. **Brotz, H., G. Bierbaum, P. E. Reynolds, and H. G. Sahl.** 1997. The lantibiotic mersacidin inhibits peptidoglycan biosynthesis at the level of transglycosylation. Eur.J.Biochem. 246:193-199.
4. **Brötz, H., M. Josten, I. Wiedemann, U. Schneider, F. Götz, G. Bierbaum, and H.-G. Sahl.** 1998. Role of lipid-bound peptidoglycan precursors in the formation of pores by nisin, epidermin and other lantibiotics. Molecular Microbiology 30:317-327. Molecular Microbiology 30:317-327.
5. **Calandra, G. B. and R. M. Cole.** 1980. Lysis and Protoplast Formation of Group B *Streptococci* by Mutanolysin. Infection and Immunity 28:1033-1037.
6. **Chopin, A., M. C. Chopin, A. Moillo-Batt, and P. Langella.** 1984. Two plasmid-determined restriction and modification systems in *Streptococcus lactis*. Plasmid 11:260-263.
7. **Crandall, A. D. and T. J. Montville.** 1998. Nisin resistance in *Listeria monocytogenes* ATCC 700302 is a complex phenotype. Applied and Environmental Microbiology 64:231-237.
8. **Davies, E. A., M. B. Falahee, and M. R. Adams.** 1996. Involvement of the cell envelope of *Listeria monocytogenes* in the acquisition of nisin resistance. Journal of Applied Microbiology 81:139-146.
9. **Delcour, J., T. Ferrain, M. Deghorian, E. Palumbo, and P. Hols.** 1999. The biosynthesis and functionality of the cell wall of lactic acid bacteria. Antonie van Leeuwenhoek 76:159-184.
10. **Delves-Broughton, J., P. Blackburn, R. J. Evans, and J. Hugenholtz.** 1996. Applications of the bacteriocin, nisin. Antonie van Leeuwenhoek 69:193-202.
11. **Dijkstra, A. J. and W. Keck.** 1996. Peptidoglycan as a barrier to transenvelope transport.
   Journal of Bacteriology 178:5555-5562.
**12. Duwat, P., A. Cochu, S. D. Ehrlich, and A. Gruss.** 1997. Characterization of *Lactococcus lactis* UV-sensitive mutants obtained by ISS1 transpostion. Journal of Bacteriology 179:4473-4479.
**13. Fisher, W.** 1994. Lipoteichoic acids and lipoglycans, p. 199-215. *In* J.M.Ghuysen and R.Hackenbeck (ed.), Bacterial Cell Wall. ESCOM Elsevier Science, Amsterdam, The Netherlands.
14. **Ghuysen, J. M.** 1994. Molecular structures of penicillin-binding proteins and beta-lactamases.
   Trends Microbiology 2:372-380.
15. **Goffin, C. and J. M. Ghuysen.** 1998. Multimodular penicillin-binding proteins: an enigmatic family of orthologs and paralogs. Microbiol.Mol.Biol.Rev. 62:1079-1093.
16. **Gravesen, A., K. Sorensen, F. M. Aarestrup, and S. Knochel.** 2001. Spontaneous nisin-resistant *Listeria monocytogenes* mutants with increased expression of a putative penicillin-binding protein and their sensitivity to various antibiotics. Microbial Drug Resistance 7:127-135.
17. **Gross, E. and J. L. Morell.** 1971. The structure of nisin. Journal of the American Chemical Society 93:4634-3635.
18. **Grossiord, B. P., E. J. Luesink, E. E. Vaughan, A. Arnaud, and W. M. de Vos.** 2003.
   Characterization, Expression and mutation of the *L lactis gal* PMKTE genes, involved in the galactose utilization via the Leloir pathway. Journal of Bacteriology 185:870-878.
19. **Guder, A., I. Wiedemann, and H.-G. Sahl.** 2000. Post-translationally modified Bacteriocins the Lantibiotics. Biopolymers 55:62-73.
20. **Holtje, J. V.** 1998. Growth of the stress-bearing and shape-maintaining murein sacculus of Escherichia coli. Microbiol.Mol.Biol.Rev. 62:181-203.
21. **Hurst, A.** 1981. Nisin. Advances in Applied Microbiology 23:85-123.
22. **Jung, G.** 1991. Lantibiotics: a survey, p. 1-34. *In* H.-G. Sahl and G. Jung (eds.), Lantibiotics: a survey. ESCOM Science Publishers, Amsterdam, The Netherlands.
23. **Kramer, N. E., E. Breukink, E. J. Smid, J. Kok, O. P. Kuipers, and B. de Kruijff.** 2003.
   Sensitivity or resistance of Gram-positive bacteria to nisin is not determined by the amount of the receptor Lipid II. to be submitted .
24. **Kramer, N. E., S. A. F. T. Hijum, J. Knol, J. Kok, and O. P. Kuipers.** 2004. Identification by DNA-microarrays of genes involved in acquired resistance against nisin in *Lactococcus lactis*. to be submitted.
25. **Lichtman, S. N.** 1992. Degradation of Endogenous Bacterial Cell Wall Polymers by the Muralytic Enzyme Mutanolysin Prevents Hepatobiliary Injury in Genetically Susceptible Rats with Experimental Intestinal Bacterial Overgrowth. Journal of Clinical Investigation 90:1313-1322.
26. **Maisnier-Patin, S. and J. Richard.** 1996. Cell wall changes in nisin-resistant variants of *Listeria innocua* grown in the presence of high nisin concentrations. FEMS Microbiology Letters **140**:29-35.
27. **Mattick, A. T. and A. Hirsch.** 1944. A powerful inhibitory substance produced by group N *streptococci.* Nature 154:551.
28. **Ming, X. and M. A. Daeschel.** 1993. Nisin resistance of foodborne bacteria and the specific resistance responses of *Listeria monocytogenes* Scott-A. Journal of Food proteins 56:944-948.
29. **Morath, S., A. Geyer, and T. Hartung.** 2001. Structure-function relationship of cytokine induction by lipoteichoic acid from *Staphylococcus aureus*. Journal Exp.Med. 193:393-397.
30. **Perkins, H. R.** 1969. Specificity of combination between mucopeptide precursors and vancomycin or ristocetin. Biochemistry Journal 111:205.
31. **Peschel, A., M. Otto, R. W. Jack, H. Kalbacher, G. Jung, and F. Götz.** 1999. Inactivation of the *dlt* operon in *Staphylococcus aureus* confers sensitivity to defensins, protegrins and other antimicrobial peptides. The Journal of Biological Chemistry 274:8405-8410.
32. **Pol, I. E. and E. J. Smid.** 1999. Combined action of nisin and carvacrol on *Bacillus cereus* and *Listeria monocytogenes*. Letters in Applied Microbiology 29:166-170.
33. **Powell, D. A., M. Duckworth, and J. Baddiley.** 1975. A membrane-associated lipomannan in mirococci. Biochemical Journal 151:387-397.
34. **Reusch, V. M. and F. C. Neuhaus.** 1971. D-alanine: membrane acceptor ligase from *Lactobacillus casei.* Journal of Biological Chemistry 246:6136-6143.
35. **Rouser, G., S. Fleisher, and A. Yamamoto.** 1970. Two dimensional then layer chromatographic separation of polar lipids and determination of phospholipids by phosphorus analysis of spots.
   Lipids 5:496.
36. **Sahl, H.-G.** 1991. Pore-formation in bacterial membranes by cationic lantibiotics., p. 347-358. *In* G. Jung and H.-G. Sahl (eds.), Nisin and Novel Lantibiotics. ESCOM Science Publishers, Amsterdam, the Netherlands.
37. **Sims, P. J., A. S. Waggoner, C. H. Wang, and J. F. Hoffman.** 1974. Studies on the mechanism by which cyanine dyes measure membrane potential in red blood cells and phosphatidylcholine vesicles. Biochemistry 13:3315-3330.
38. **Steen, A., G. Buist, K. J. Leenhouts, M. Khattabi, F. Grijpstra, A. L. Zomer, G. Venema, O. P. Kuipers, and J. Kok.** 2003. Cell wall attachment of a widely distributed peptidoglycan binding domain is hindered by cell wall constituents. Journal of Biological Chemistry 278:23874-23881.
39. **Sutcliffe, I. C. and N. Shaw.** 1991. Atypical lipoteichoic acids of Gram-positive bacteria. Journal of Bacteriology 173:7065-7069.
40. **van Heijenoort, J.** 2001. Formation of the glycan chains in the synthesis of bacterial peptidoglycan. Glycobiology 11:25R-36R.
41. **van Kraaij, C.** 1999. Utrecht University. Probing the membrane activity of nisin by protein engineering.
42. **Wiedemann, I., E. Breukink, C. van Kraaij, O. P. Kuipers, G. Bierbaum, B. de Kruijff, and H.-G. Sahl.** 2001. Specific binding of nisin to the peptidoglycan precursor lipid II combines pore formation and inhibition of cell wall biosynthesis for potent antibiotic activity. Journal of Biological Chemistry 276:1772-1779.

## Claims

1. A method for selecting a gene involved with nisin-resistance in a species of bacteria comprising providing candidate genes that are differentially expressed in nisin-resistant and sensitive strains of said species and selecting from said candidate genes at least one gene that results in an altered nisin-resistance phenotype in a strain of said species, when expression of said gene is altered.

2. A method according to claim 1, wherein differential expression of genes is determined by means of nucleic acid array technology.

3. A method according to claim 1 or claim 2, wherein the involvement of a candidate gene in nisin-resistance is determined by determining that the nisin-resistance phenotype of a strain of said species changes when expression of said gene in said strain is altered.

4. A method according to claim 3, wherein altering expression comprises knocking out expression of said gene or over-expressing said gene in said strain.

5. A method according to any one of claims 1-4, wherein said candidate genes comprises a gene that is the homologue of a *Lactococcus lactis* gene depicted in table 1 or table 2.

6. A method according to any one of claims 1-5, wherein a gene involved with nisin-resistance in a species of bacteria comprises a gene that is the homologue of a *Lactococcus lactis* gene depicted in table 1 or table 2.

7. A method according to claim 5 or claim 6, wherein said gene is a *Lactococcus lactis* gene.

8. A method according to any one of claims 1-7, further comprising producing a bacterial strain wherein the expression of a gene product of a gene involved in nisin-resistance in said strain is altered.

9. A method according to claim 8, wherein said bacterial strain comprises an enhanced nisin-resistance phenotype.

10. A bacterial strain obtainable by a method according to claim 8 or 9.

11. A bacterial strain according to claim 10, wherein the expression of a gene product is altered of a gene that is the homologue of a *Lactococcus lactis* gene depicted in table 1 or table 2.

12. A bacterial strain according to claim 10 or claim 11, wherein said strain is a strain that is used in the production of a food product.

13. A bacterial strain according to any one of claims 10-12, wherein said strain is a strain that is used in the production of dairy product.

14. A bacterial strain according to any one of claims 10-13, wherein said strain is a strain that is used in the production of a fermented milk product.

15. A bacterial strain according to any one of claims 10-14, wherein said dairy product is cheese, yoghurt or buttermilk.

16. A bacterial strain according to any one of claims 10-15, wherein said strain is a *Lactococcus lactis.*

17. A starter culture for fermenting a dairy precursor product comprising a bacterial strain according to any one of claims 10-16, 32.

18. A starter culture according to claim 17, comprising nisin-resistant bacterial strains, wherein at least one of said nisin-resistant strains is a bacterial strain according to any one of claims 10-16, 32.

19. A method for fermenting a dairy precursor product comprising providing said dairy precursor product with a bacterial strain according to any one of claims 10-16, 32, or a starter culture according to claim 17 or claim 18.

20. A method according to claim 19, further comprising providing said precursor dairy product with nisin.

21. A method according to claim 20, wherein said nisin is produced by a strain according to any one of claims 10-16, 32, or a starter culture according to claim 17 or claim 18.

22. A method according to claim 21, wherein said bacterial strain producing nisin over-produces said nisin.

23. A method according to any one of claim 19-22, wherein wherein said dairy precursor product is used in the production of a food product.

24. A method according to any one of claim 19-23, wherein wherein said dairy precursor product is used in the production of a dairy product.

25. A method according to any one of claim 19-24, wherein wherein said dairy precursor product is used in the production of a fermented milk product.

26. A method according to any one of claims 19-25, wherein said dairy precursor product is used in the production of cheese, yoghurt or buttermilk.

27. A method according to claim 26, further comprising producing cheese, yoghurt or buttermilk.

28. A cheese obtainable by a method according to claim 27.

29. A cheese according to claim 28, comprising a bacterial strain according to any one of claims 10-16, 32, or a debris thereof.

30. A gouda, cheddar or emmenthaler cheese according to claim 28 or claim 29.

31. A method for selecting a nisin resistant gram positive bacterial strain comprising detecting expression of a gene that is the homologue of a *Lactococcus lactis* gene depicted in table 1 or table 2 in a gram positive bacterial strain and selecting said strain when expression of said gene is indicative for nisin resistance.

32. A bacterial strain selected for nisin-resistance on the basis of the expression of a gene that is the homologue of a *Lactococcus lactis* gene depicted in table 1 or table 2.

33. A culture comprising a nisin resistant bacterial strain of a species obtained by a method according to claim 31, or a bacterial strain of a species according to claim 32, wherein said culture comprises nisin in a concentration that is toxic for nisin sensitive strains of said species.
